# EUROPEAN PATENT SPECIFICATION

(11) **EP 1 551 403 B1**
(45) Date of publication and mention of the grant of the patent: **29.04.2009**
(21) Application number: 03808198.0
(22) Date of filing: 09.10.2003
(51) Int. Cl.: A61K 31/45, C07D 231/16, C07D 231/14

(54) **5-SUBSTITUTED 2H-PYRAZONE-3-CARBOXYLIC ACID DERIVATIVES AS ANTILIPOLYTIC AGENTS FOR THE TREATMENT OF METABOLIC-RELATED DISORDERS SUCH AS DYSLIPIDEMIA**
5-SUBSTITUIERTE 2H-PYRAZON-3-CARBONSÄURE-DERIVATE ALS ANTILIPOLYTISCHE MITTEL ZUR BEHANDLUNG VON STOFFWECHSELSTÖRUNGEN, WIE Z.B. DYSLIPIDEMIE
DERIVES D'ACIDE 2H-PYRAZOLE-3-CARBOXYLIQUE SUBSTITUE EN 5 UTILISES EN TANT QU'AGENTS ANTILIPOTYQUES POUR LE TRAITEMENT DE TROUBLES METABOLIQUES, TELS QUE LA DYSLIPIDEMIE

(30) Priority: 10.10.2002 US 418057 P
(43) Date of publication of application: 13.07.2005
(73) Proprietor: Arena Pharmaceuticals, Inc., San Diego, CA 92121-3223 (US)
(72) Inventor: SEMPLE, Graeme, San Diego, CA 92127 (US); AVERBUJ, Claudia, San Diego, CA 92130 (US); SKINNER, Philip, San Diego, CA 92109 (US); GHARBAOUI, Tawfik, Escondido, CA 92029 (US); SHIN, Young-Jun, San Diego, CA 92126 (US)
(74) Representative: Wytenburg, Wilhelmus Johannes
(86) International application number: PCT/US2003/032174
(87) International publication number: WO 2004/032928

(56) References cited:
- GB-A- 1 048 104
- US-A- 3 980 646

## Description

### FIELD OF THE INVENTION

The present invention relates to certain pyrazole carboxylic acid derivatives, and pharmaceutically acceptable salts thereof, which exhibit useful pharmacological properties, for example as agonists for the receptor **RUP25**. Also provided by the present invention are pharmaceutical compositions containing compounds of the invention, and use of the compounds of the invention for the preparation of a medicament for the treatment of metabolic-related disorders, including dyslipidemia, atherosclerosis, coronary heart disease, insulin resistance, type 2 diabetes, Syndrome-X and the like. In addition, the present invention also provides for pharmaceutical compositions in combination with other active agents, for example, those agents belonging to the class of α-glucosidase inhibitors, aldose reductase inhibitors, biguanides, HMG-CoA reductase inhibitors, squalene synthesis inhibitors, fibrates, LDL catabolism enhancers, angiotensin converting enzyme (ACE) inhibitors, insulin secretion enhancers, thiazolidinedione and the like.

### BACKGROUND OF THE INVENTION

### Compounds of the invention as Antilipolytic Agents

Atherosclerosis and stroke are the numbers one and number three leading causes of death of both men and women in the United States. Type 2 diabetes is a public health problem that is serious, widespread and increasing. Elevated levels of low density lipoprotein (LDL) cholesterol or low levels of high density lipoprotein (HDL) cholesterol are, independently, risk factors for atherosclerosis and associated cardiovascular pathologies. In addition, high levels of plasma free fatty acids are associated with insulin resistance and type 2 diabetes. One strategy for decreasing LDL-cholesterol, increasing HDL-cholesterol, and decreasing plasma free fatty acids is to inhibit lipolysis in adipose tissue. This approach involves regulation of hormone sensitive lipase, which is the rate-limiting enzyme in lipolysis. Lipolytic agents increase cellular levels of cAMP, which leads to activation of hormone sensitive lipase within adipocytes. Agents that lower intracellular cAMP levels, by contrast, would be antilipolytic.

It is also worth noting in passing that an increase in cellular levels of cAMP down-regulates the secretion of adiponectin from adipocytes [Delporte, ML et al. Biochem J (2002) July]. Reduced levels of plasma adiponectin have been associated with metabolic-related disorders, including atherosclerosis, coronary heart disease, insulin resistance and type 2 diabetes [Matsuda, M et al. J Biol Chem (2002) July and reviewed herein].

Nicotinic acid (niacin, pyridine-3-carboxylic acid) is a water-soluble vitamin required by the human body for health, growth and reproduction; a part of the Vitamin B complex. Nicotinic acid is also one of the oldest used drugs for the treatment of dyslipidemia. It is a valuable drug in that it favorably affects virtually all of the lipid parameters listed above [Goodman and Gilman's Pharmacological Basis of Therapeutics, editors Harmon JG and Limbird LE, Chapter 36, Mahley RW and Bersot TP (2001) pages 971-1002]. The benefits of nicotinic acid in the treatment or prevention of atherosclerotic cardiovascular disease have been documented in six major clinical trials [Guyton JR (1998) Am J Cardiol 82:18U-23U] Nicotinic acid and related derivatives, such as, acipimox have recently been discussed [Lorenzen, A et al (2001) Molecular Pharmacology 59:349-357].

Nicotinic acid inhibits the production and release of free fatty acids from adipose tissue, likely via an inhibition of adenylyl cyclase, a decrease in intracellular cAMP levels, and a concomitant decrease in hormone sensitive lipase activity. Agonists that down-regulate hormone sensitive lipase activity leading to a decrease in plasma free fatty acid levels are likely to have therapeutic value. The consequence of decreasing plasma free fatty acids is two-fold. First, it will ultimately lower LDL-cholesterol and raise HDL-cholesterollevels, independent risk factors, thereby reducing the risk of mortality due to cardiovascular incidence subsequent to atheroma formation. Second, it will provide an increase in insulin sensitivity in individuals with insulin resistance or type 2 diabetes. Unfortunately, the use of nicotinic acid as a therapeutic is partially limited by a number of associated, adverse side-effects. These include flushing, free fatty acid rebound, and liver toxicity.

The rational development of novel, nicotinic acid receptor agonists that have fewer side-effects will be valuable, but to date this has been hindered by the inability to molecularly identify the nicotinic acid receptor. Furthermore, other receptors of the same class may exist on the surface of adipocytes and similarly decrease hormone sensitive lipase activity through a reduction in the level of intracellular cAMP but without the elicitation of adverse effects such as flushing, thereby representing promising novel therapeutic targets. Recent work suggests that nicotinic acid probably acts through a specific GPCR [Lorenzen A, et al. (2001) Molecular Pharmacology 59:349-357 and reviewed therein].

US Patent No 3,980,646 (Brenner et al.) describes certain 3-methylpyrazole-5-carboxylates which apparently have lipolysis-inhibiting activity, and apparently are useful in reducing triglyceride and cholesterol levels. The broadest definition of the compounds is shown below:

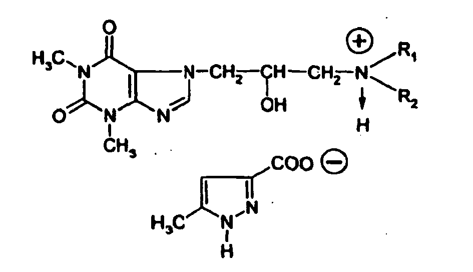

The anion is the carboxylate corresponding to 5-methyl-1 H-pyrazole-3-carboxylic acid (conveniently denoted "3-C-5-MP").

GB Patent No 1,048,104 (Upjohn Company) describes certain 3,5-disubstituted pyrazoles which apparently have hypoglycemic activity and apparently are useful in the management of human diabetes and hypercholesteremia and in the treatment of ketosis in children. The broadest definition of the compounds is shown below: 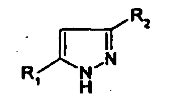 wherein R₁ is alkyl and R₂ is carboxyl, alkoxycarbonyl, carbamoyl, or alkyl-monosubstituted carbamoyl, wherein the alkyl groups contain 1 to 4 carbon atoms. The only examples are 5-methyl-1 H-pyrazole-3-carboxylic acid (conveniently denoted "3-C-5-MP") and 5-ethyl-1 H-pyrazole-3-carboxylic acid (conveniently denoted "3-C-5-EP").

### SUMMARY OF THE INVENTION

One aspect of the present invention encompasses certain pyrazole carboxylic acid derivatives as shown in Formula (Ia): 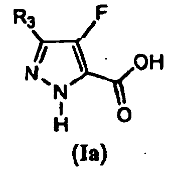 wherein:
R₃ is C₃₋₆ cycloalkyl, C₁₋₁₂ alkyl, C₁₋₁₂ haloalkyl, C₃₋₆ cycloalkyl-C₁₋₄-alkylene, aryl-C₁₋₄-alkylene or heteroaryl-C₁₋₄-alkylene, wherein said aryl-C₁₋₄-alkylene and heteroaryl-C₁₋₄-alkylene can be optionally substituted with 1 to 5 substituents selected from the group consisting of C₁₋₄ acyl, C₁₋₄ acyloxy, C₂₋₄ alkenyl, C₁₋₄ alkoxy, C₁₋₄ alkyl, C₁₋₄ alkylcarboxamide, C₂₋₄ alkynyl, C₁₋₄ alkylsulfonamide, C₁₋₄ alkylsulfinyl, C₁₋₄ alkylsulfonyl, C₁₋₄ alkylthio, C₁₋₄ alkylureyl, amino, C₁₋₄ alkylamino, C₁₋₄ dialkylamino, arylsulfonyl, carbo-C₁₋₄-alkoxy, carboxamide, carboxy, cyano, C₃₋₆ cycloalkyl, C₁₋₄ dialkylcarboxamide, C₁₋₄ dialkylsulfonamide, halogen, C₁₋₄ haloalkoxy, C₁₋₄ haloalkyl, C₁₋₄ haloalkylsulfinyl, C₁₋₄ haloalkylsulfonyl, C₁₋₄ haloalkylthio, heterocyclyl, hydroxyl, thio, nitro, C₄₋₆ oxo-cycloalkyl, sulfonamide and sulfonic acid; or
   a pharmaceutically acceptable salt, solvate or hydrate thereof.

Another aspect of the present invention encompasses compounds of the group consisting of 4-Fluoro-5-methyl-2*H*-pyrazole-3-carboxylic acid; 5-Difluoromethyl-4-fluoro-2*H*-pyrazole-3-carboxylic acid; 5-(2,2-Difluoro-ethyl)-fluoro-2*H*-pyrazole-3-carboxyilic acid; 5-(1,1-Difluoro-ethyl)-4-fluoro-2H-pyrazole-3-carboxylic acid; 5-(2,2-Difluoro-propyl)-4-fluoro-2*H*-pyrazole-3-carboxylic acid; 5-Ethyl-4-fluoro-2*H*-pyrazole-3-carboxylic acid; 4-Fluoro-5-propyl-2*H* pyrazole-3-carboxylic acid; 5-Butyl-4-fluoro-2*H*-pyrazole-3-carboxylic acid; 4-Fluoro-5-pentyl-2*H*-pyrazole-3-carboxylic acid; 4-Fluoro-5-hexyl-2*H*-pyrazole-3-carboxylic acid; 4-Fluoro-5-heptyl-2*H*-pyrazole-3-carboxylic acid; 4-Fluoro-5-octyl-2*H*-pyrazole-3-carboxylic acid; 4-Fluoro-5-nonyl-2*H*-pyrazole-3-carboxylic acid; 5-Decyl-4-fluoro-2*H*-pyrazole-3-carboxylic acid; 4-Fluoro-5-undecyl-2*H*-pyrazole-3-carboxylic acid; 5-Dodecyl-4-fluoro-2*H*-pyrazole-3-carboxylic acid; 5-cyclopropyl-4-fluoro-2*H*-pyrazole-3-carboxylic acid and 5-Cyclopropylmethyl-4-fluoro-2*H*-pyrazole-3-carboxylic acid.

Another aspect of the present invention encompasses certain pharmaceutical compositions comprising a pharmaceutically acceptable carrier and a compound of Formula **(Ia).**

Another aspect of the present invention encompasses certain pharmaceutical compositions comprising a pharmaceutically acceptable carrier and a compound shown in Formula (Ia): 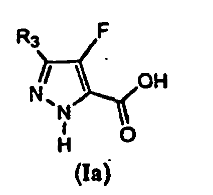 wherein:
R₃ is C₃₋₆ cycloalkyl, C₁₋₁₂ alkyl, C₁₋₁₂ haloalkyl, C₃₋₆ cycloalkyl-C₁₋₄-alkylene, aryl-C₁₋₄-alkylene or heteroaryl-C₁₋₄-alkylene, wherein said aryl-C₁₋₄-alkylene and heteroaryl-C₁₋₄ -alkylene can be optionally substituted with 1 to 5 substituents selected from the group consisting of C₁₋₄ acyl, C₁₋₄ acyloxy, C₂₋₄ alkenyl, C₁₋₄ alkoxy, C₁₋₄ alkyl, C₁₋₄ alkylcarboxamide, C₂₋₄ alkynyl, C₁₋₄ alkylsulfonamide, C₁₋₄ alkylsulfinyl, C₁₋₄ alkylsulfonyl, C₁₋₄ alkylthio, C₁₋₄ alkylureyl, amino, C₁₋₄ alkylamino, C₁₋₄ dialkylamino, arylsulfonyl, carbo-C₁₋₄-alkoxy, carboxamide, carboxy, cyano, C₃₋₆ cycloalkyl, C₁₋₄ dialkylcarboxamide, C₁₋₄ dialkylsulfonamide, halogen, C₁₋₄ haloalkoxy, C₁₋₄ haloalkyl, C₁₋₄ haloalkylsulfinyl, C₁₋₄ haloalkylsulfonyl, C₁₋₄ haloalkylthio, heterocyclyl, hydroxyl, thio, nitro, C₄₋₆ oxo-cycloalkyl, sulfonamide and sulfonic acid, or
   a pharmaceutically acceptable salt, solvate or hydrate thereof.

Another aspect of the present invention encompasses pharmaceutical compositions, as described herein, further comprising one or more agent selected from the group consisting of α-glucosidase inhibitor, aldose reductase inhibitor, biguanide, HMG-CoA reductase inhibitor, squalene synthesis inhibitor, fibrate, LDL catabolism enhancer, angiotensin converting enzyme inhibitors, insulin secretion enhancer and thiazolidinedione.

Described herein are methods of modulating a **RUP25** receptor comprising contacting the receptor with an effective amount of a compound as described herein. In some embodiments, the compound is an agonist of said receptor.

Described herein are methods of modulating a **RUP25** receptor in an individual comprising contacting the receptor with an effective amount of a compound as described herein. In some embodiments, the modulation treats a metabolic-related disorder.

Described herein are methods of modulating **RUP25** receptor function in a cell, tissue or individual comprising contacting the cell, tissue or individual with an effective amount of a compound as described herein. In some embodiments, the **RUP25** receptor function is associated with a metabolic-related disorder.

Described herein are methods of treatment of a metabolic-related disorder comprising administering to an individual in need of such treatment a therapeutically effective amount of a compound or a pharmaceutical composition as described herein.

In some embodiments of the present invention, the metabolic-related disorder is selected from the group consisting of dyslipidemia, atherosclerosis, coronary heart disease, insulin resistance, obesity, impaired glucose tolerance, atheromatous disease, hypertension, stroke, Syndrome X, heart disease and type 2 diabetes. In some embodiments, the metabolic-related disorder is selected from the group consisting of dyslipidemia, atherosclerosis, coronary heart disease, insulin resistance and type 2 diabetes.

In some embodiments of the present invention, the individual is a mammal. In some embodiments, the mammal is a human.

Another aspect of the present invention encompasses methods of producing a pharmaceutical composition comprising admixing a compound as described herein and a pharmaceutically acceptable carrier.

Another aspect of the present invention encompasses the use of compounds of Formula (Ia):
for the manufacture of a medicament for use in the treatment of a metabolic-related disorder.

Another aspect of the present invention encompasses compounds as described herein for the manufacture of a medicament for use in the treatment of a metabolic-related disorder further comprising an agent selected from the group consisting of α-glucosidase inhibitor, aldose reductase inhibitor, biguanide, HMG-CoA reductase inhibitor, squalene synthesis inhibitor, fibrate, LDL catabolism enhancer, angiotensin converting enzyme inhibitor, insulin secretion enhancer and thiazolidinedione.

Another aspect of the present invention is a compound according to any of the embodiments described herein or a pharmaceutical composition as described herein for use in a method of treatment of the human or animal body by therapy.

Another aspect of the present invention is a compound according to any of the embodiments described herein or a pharmaceutical composition as described herein for use in a method of treatment of a metabolic-related disorder of the human or animal body by therapy.

These and other aspects of the invention disclosed herein will be set forth in greater detail as the patent disclosure proceeds.

### DETAILED DESCRIPTION OF THE INVENTION

### DEFINITIONS:

The scientific literature has adopted a number of terms, for consistency and clarity, the following definitions will be used throughout this patent document.

**AGONISTS** shall mean moieties that interact and activate the receptor, such as the **RUP25** receptor and initiate a physiological or pharmacological response characteristic of that receptor. For example, when moieties activate the intracellular response upon binding to the receptor, or enhance GTP binding to membranes.

**AMINO ACID ABBREVIATIONS** used herein are set out in TABLE 1:

| TABLE 1 | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| METHIONINE | MET | M |
| PHENYLALANINE | PHE | F |
| PROLINE | PRO | P |
| SERENE | SER | S |
| THREONINE | THR | T |
| TRYPTOPHAN | TRP | W |
| TYROSINE | TYR | Y |
| VALINE | VAL | V |

The term **ANTAGONISTS** is intended to mean moieties that competitively bind to the receptor at the same site as agonists (for example, the endogenous ligand), but which do not activate the intracellular response initiated by the active form of the receptor, and can thereby inhibit the intracellular responses by agonists or partial agonists. Antagonists do not diminish the baseline intracellular response in the absence of an agonist or partial agonist.

**ATHEROSCLEROSIS** is intended herein to encompass disorders of large and medium-sized arteries that result in the progressive accumulation within the intima of smooth muscle cells and lipids.

### CHEMICAL GROUP, MOIETY OR RADICAL:

The term **"C₁₋₄ acyl"** denotes a C₁₋₄ alkyl radical attached to a carbonyl wherein the definition of alkyl has the same definition as described herein; some examples include but not limited to, acetyl, propionyl, n-butanoyl, iso-butanoyl, sec-butanoyl, t-butanoyl (i.e., pivaloyl), pentanoyl and the like.

The term **"C₁₋₄ acyloxy"** denotes an acyl radical attached to an oxygen atom wherein acyl has the same definition has described herein; some examples include but not limited to acetyloxy, propionyloxy, butanoyloxy, *iso*-butanoyloxy, sec-butanoyloxy, t-butanoyloxy and the like.

The term **"C₂₋₄ alkenyl"** denotes a radical containing 2 to 4 carbons wherein at least one carbon-carbon double bond is present, some embodiments are 2 to 3 carbons, and some embodiments have 2 carbons. Both *E* and *Z* isomers are embraced by the term **"alkenyl."** Furthermore, the term **"alkenyl"** includes di-enes. Accordingly, if more than one double bond is present, then the bonds may be all *E* or *Z* or a mixture of *E* and *Z*. Examples of an alkenyl include vinyl, propenyl, allyl, isopropenyl, 2-methyl-propenyl 1-methyl-propenyl, but-1-enyl, but-2-enyl, but-3-enyl, buta-1,3-dienyl, and the like.

The term **"C₁₋₄ alkoxy"** denotes an alkyl radical, as defined herein, attached directly to an oxygen atom Examples include methoxy, ethoxy, n-propoxy, iso-propoxy, n-butoxy, t-butoxy, iso-butoxy, sec-butoxy and the like.

The term **"alkyl"** denotes a straight or branched carbon radical containing the number of carbons, for examples, in some embodiments, alkyl is **"C₁₋₁₂ alkyl"** and the group contains 1 to 12 carbons, some embodiments contain 1 to 10 carbons, some embodiments contain 1 to 8 carbons, some embodiments contain 1 to 6 carbons, some embodiments contain 1 to 4 carbons (i.e., **"C₁₋₄ alkyl"**), some embodiments are 1 to 3 carbons, and some embodiments are 1 or 2 carbons. Examples of an alkyl include, but not limited to, methyl, ethyl, n-propyl, iso-propyl, n-butyl, iso-butyl, t-butyl, sec-butyl, n-pentyl, iso-pentyl, sec-pentyl, neo-pentyl, pent-3-yl, 2-methyl-but-1-yl, 1,2-dimethyl-prop-1-yl, n-hexyl, iso-hexyl, sec-hexyl, neo-hexyl, 1-ethyl-2-methyl-prop-1-yl, 1,2,2-trimethyl-prop-1-yl, 1,1,2-trimethyl-prop-1-yl, 1-ethyl-1-methyl-prop-1-yl, 1,1-dimethyl-but-1-yl, 1,2-dimethyl-but-1-yl, 2,3-dimethyl-but-1-yl, 2,2-dimethyl-but-1-yl, 1,3-dimethyl-but-1-yl, hex-3-yl, 2-methyl-pent-1-yl, 3-methyl-pent-1-yl, heptyl,1-methyl-hexyl, 1-ethyl-pentyl. 1-propyl-butyl,octyl,1-methyl-heptyl, 1-ethyl-hexyl, 1-propyl-pentyl, nonyl, 1-methyl-octyl, 1-ethyl-heptyl, 1-propyl-hexyl, 1-Butyl-pentyl, decyl, undecyl, dodecyl, and the like.

The term **"C₁₋₄ alkylcarboxamido"** or **"C₁₋₄ alkylcarboxamide"** denotes a single C₁₋₄ alkyl group attached to the nitrogen or carbonyl of an amide group, wherein alkyl has the same definition as found herein. The C₁₋₄ alkylcarboxamide may be represented by the following: 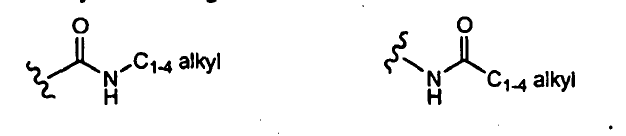 Examples include, but not limited to, *N*-methylcarboxamide, *N*-ethylcarboxamide, *N*-n-propylcarboxamide, *N*-iso-propylcarboxamide, *N*-n-butylcarboxamide, *N*-sec-butylcarboxamide, *N*-iso-butylcarboxamide, *N*-t-butylcarboxamide and the like.

The term **"C₁₋₄ alkylene"** denotes a C₁₋₄ divalent straight carbon group. In some embodiments C₁₋₄ alkylene refers to, for example, -CH₂, -CH₂CH₂-, -CH₂CH₂CH₂-, and -CH₂CH₂CH₂CH₂-.

The term **"C₁₋₄ alkylsulfinyl"** denotes a C₁₋₄ alkyl radical attached to a sulfoxide radical of the formula: -S(O)- wherein the alkyl radical has the same definition as described herein. Examples include, but not limited to, methylsulfinyl, ethylsulfinyl, n-propylsulfinyl, iso-propylsulfinyl, n-butylsulfinyl, sec-butylsulfinyl, iso-butylsulfinyl, t-butyl, and the like.

The term **"C₁₋₄ alkylsulfonamide"** refers to the groups 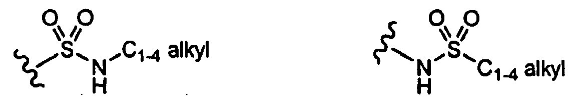 wherein C₁₋₄ alkyl has the same definition as described herein.

The term **"C₁₋₄ alkylsulfonyl"** denotes a C₁₋₄ alkyl radical attached to a sulfone radical of the formula: -S(O)₂- wherein the alkyl radical has the same definition as described herein. Examples include, but not limited to, methylsulfonyl, ethylsulfonyl, n-propylsulfonyl, iso-propylsulfonyl, n-butylsulfonyl, sec-butylsulfonyl, iso-butylsulfonyl, t-butyl, and the like.

The term **"C₁₋₄ alkylthio"** denotes a C₁₋₄ alkyl radical attached to a sulfide group of the formula: -S- wherein the alkyl radical has the same definition as described herein. Examples include, but not limited to, methylsulfanyl (i.e., CH₃S-), ethylsulfanyl, n-propylsulfanyl, iso-propylsulfanyl, n-butylsulfanyl, sec-butylsulfanyl, iso-butylsulfanyl, t-butyl, and the like.

The term **"C₁₋₄ alkylureyl"** denotes the group of the formula: -NC(O)N-wherein one are both of the nitrogens are substituted with the same or different C₁₋₄ alkyl group wherein alkyl has the same definition as described herein. Examples of an alkylureyl include, but not limited to, CH₃NHC(O)NH-, NH₂C(O)NCH₃-, (CH₃)₂N(O)NH-, (CH₃)₂N(O)NH-, (CH₃)₂N(O)NCH₃-, CH₃CH₂NHC(O)NH-, CH₃CH₂NHC(O)NCH₃-, and the like.

The term **"C₂₋₄ alkynyl"** denotes a radical containing 2 to 4 carbons and at least one carbon-carbon triple bond, some embodiments are 2 to 3 carbons, and some embodiments have 2 carbons. Examples of an alkynyl include, but not limited to, ethynyl, prop-1-ynyl, 3-prop-2-ynyl, but-1-ynyl, 1-methyl-prop-2-ynyl, buta-1,3-diynyl, and the like. The term **"alkynyl"** includes di-ynes.

The term **"amino"** denotes the group -NH₂.

The term **"C₁₋₄ alkylamino"** denotes one alkyl radical attached to an amino radical wherein the alkyl radical has the same meaning as described herein. Some examples include, but not limited to, methylamino, ethylamino, n-propylamino, iso-propylamino, n-butylamino, sec-butylamino, iso-butylamino, t-butylamino, and the like. Some embodiments are **"C₁₋₂ alkylamino."**

The term "aryl" denotes an aromatic ring radical containing 6 to 10 ring carbons. Examples include phenyl and naphthyl.

The term **"aryl-C₁₋₄-alkylene"** denotes an aryl directly bonded to a C₁₋₄-alkylene group, wherein both definitions have the same meaning as described herein. Examples of aryl-C₁₋₄-alkylene include but not limited to, benzyl, 2-phenyl-ethyl, 3-phenyl-propyl, naphthylmethyl, 2-napthyl-ethyl, 3-napthyl-propyl, and the like.

The term **"carbo-C₁₋₄-alkoxy"** denotes a C₁₋₄ alkyl ester of a carboxylic acid, wherein the alkyl group is as defined herein. Examples include, but not limited to, carbomethoxy, carboethoxy, carbopropoxy, carboisopropoxy, carbobutoxy, carbo-sec-butoxy, carbo-iso-butoxy, carbo-t-butoxy, and the like.

The term **"carboxamide"** refers to the group **-CONH₂.**

The term **"carboxy"** or **"carboxyl"** denotes the group **-CO₂H;** also referred to as a carboxylic acid group.

The term "cyano" denotes the group -**CN**.

The term **"C₃₋₆ cycloalkyl"** denotes a saturated ring radical containing 3 to 6 carbons; some embodiments contain 3 to 5 carbons; some embodiments contain 3 to 4 carbons. Examples include, cyclopropyl, cyclobutyl, cyclopentyl, cyclohexyl.

The term "C₃₋₆ **cycloalkyl-C₁₋₄-alkylene"** denotes a C₃₋₆ cycloalkyl directly bonded to a C₁₋₄-alkylene group, wherein both definitions have the same meaning as described herein. Examples of C₃₋₆ cycloalkyl-C₁₋₄-alkylene include, but are not limited to, cyclopropylmethyl, cyclobutylmethyl, cyclopentylmethyl, cyclohexylmethyl, 2-cyclopropyl-ethyl, 2-cyclobutyl-ethyl, 2-cyclopentyl-ethyl, 2-cyclohexyl-ethyl, 3-cyclopropyl-propyl, 3-cyclobutyl-propyl, and the like.

The term **"C₁₋₄ dialkylamino"** denotes an amino substituted with two of the same or different alkyl radicals wherein alkyl radical has the same definition as described herein. A C₁₋₄ dialkylamino may be represented by the following group: 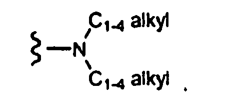 Examples of C₁₋₄ dialkylamino include, but not limited to, dimethylamino, methylethylamino, diethylamino, methylpropylamino, methylisopropylamino, and the like.

The term **"C₁₋₄ dialkylcarboxamido"** or **"C₁₋₄ dialkylcarboxamide**" denotes two alkyl radicals, that are the same or different, attached to an amide group, wherein alkyl has the same definition as described herein. A C₁₋₄ dialkylcarboxamide may be represented by the following groups: 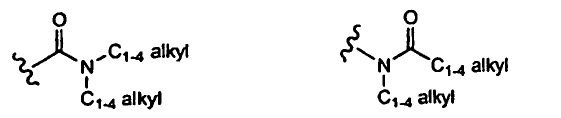 wherein C₁₋₄ alkyl has the same definition as described herein. Examples of a dialkylcarboxamide include, but not limited to, *N,N*-dimethylcarboxamide, *N*-methyl-*N-*ethylcarboxamide, *N,N*-diethylcarboxamide, *N*-methyl-*N*-isopropylcarboxamide, and the like.

The term **"C₁₋₄ dialkylsulfonamide"** refers to one of the following groups shown below: 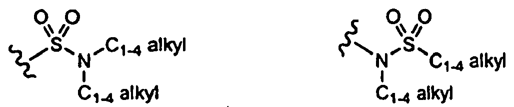 wherein C₁₋₄ has the same definition as described herein.

The term **"C₁₋₄ haloalkoxy"** denotes a haloalkyl, as defined herein, which is directly attached to an oxygen atom. Examples include, but not limited to, difluoromethoxy, trifluoromethoxy, 2,2,2-trifluoroethoxy, pentafluoroethoxy and the like.

The term **"haloalkyl"** denotes an alkyl group wherein the alkyl is substituted with halogen ranging from one to fully substituted, wherein a fully substituted haloalkyl can be represented by the formula CₕL₂ₕ₊₁ wherein L is a halogen and "h" represents the number of carbon atoms; when more than one halogen is present then the halogens may be the same or different and selected from the group consisting of F, Cl, Br and I; it is understood that the terms "alkyl" and "halogen" have the same definition as found herein. In some embodiments, haloalkyl is a **"C₁₋₁₂ haloalkyl"** and the group contains 1 to 12 carbons, some embodiments contain 1 to 10 carbons, some embodiments contain 1 to 8 carbons, some embodiments contain 1 to 6 carbons, some embodiments contain 1 to 4 carbons (i.e., **"C₁₋₄ haloalkyl"**), some embodiments are 1 to 3 carbons, and some embodiments are 1 or 2 carbons. When the haloalkyl is fully substituted with halogen atoms, this group is referred to herein as a perhaloalkyl, one example, is an alkyl fully substituted with fluorine atoms and is referred to herein as a **"perfluoroalkyl."** In some embodiments, examples of a haloalkyl include, but not limited to, difluoromethyl, fluoromethyl, 2,2,2-trifluoro-ethyl, 2,2-difluoro-ethyl, 2-fluoro-ethyl, 1,2,2,2-tetrafluoroethyl, 1,2,2-trifluoro-ethyl, 1,2-difluoro-ethyl, 1,1-difluoro-ethyl, 1,1,2,2-tetrafluoroethyl, 1,1,2-trifluorocthyl, 3,3,3-trifluoro-propyl, 2,2-difluoro-propyl, 3,3-difluoro-propyl, 3-fluoro-propyl, 2,3,3,3-tetrafluoro-propyl, 2,3,3-trifluoro-propyl, 2,3-Difluoro-propyl, 2,2,3,3,3-pentafluoro-propyl, 2,2,3,3-tetrafluoro-propyl, 2,2,3-trifluoro-propyl, 1,2,3,3,3-pentafluoro-propyl,1,2,3,3-tetrafluoro-propyl, 1,2,3-trifluoro-propyl, 3,3-difluoro-propyl, 1,2,2,3,3-pentafluoro-propyl, 1,2,2,3-tetrafluoro-propyl, 1,1,2,2,3,3-hexafluoro-propyl, 1,1,2,2,3-pentafluoro-propyl, 4,4-difluoro-butyl, 3,3-difluoro-butyl, 4,4,4-trifluoro-butyl, 3,3-difluoro-butyl, 5,5,5-trifluoro-pentyl, 4,4-difluoro-pentyl, 6,6,6-trifluoro-hexyl, 5,5-difluoro-hexyl, and the like. In some embodiments, examples of a perfluoroalkyl include, but not limited to, trifluoromethyl, pentafluoroethyl, heptafluoropropyl, 1,2,2,2-tetrafluoro-1-trifluoromethyl-ethyl, and the like.

The term **"C₁₋₄ haloalkylsulfinyl"** denotes a haloalkyl radical attached to a sulfoxide group of the formula: -S(O)- wherein the haloalkyl radical has the same definition as described herein.

The term **"C₁₋₄ haloalkylsulfonyl"** denotes a haloalkyl radical attached to a sulfone group of the formula: -S(O)₂- wherein haloalkyl has the same definition as described herein.

The term **"C₁₋₄ haloalkylthio"** denotes a haloalkyl radical directly attached to a sulfur atom wherein the haloalkyl has the same meaning as described herein.

The term **"halogen"** or "halo" denotes to a fluoro, chloro, bromo or iodo group.

The term **"heteroaryl"** denotes an aromatic ring system that may be a single ring, two fused rings or three fused rings wherein at least one ring carbon is replaced with a heteroatom selected from, but not limited to, the group consisting of O, S and N wherein the N can be optionally substituted with H, C₁₋₄ acyl or C₁₋₄ alkyl. Examples of heteroaryl groups include, but not limited to, pyridyl, benzofuranyl, pyrazinyl, pyridazinyl, pyrimidinyl, triazinyl, quinoline, benzoxazole, benzothiazole, 1*H-*benzimidazole, isoquinoline, quinazoline, quinoxaline and the like. In some embodiments, the heteroaryl atom is O, S, NH, examples include, but not limited to, pyrrole, indole, and the like. Other examples include, but are not limited to, those in TABLE 2 and the like.

The term **"heteroaryl-C₁₋₄alkylene"** denotes a heteroaryl group directly bonded to a C₁₋₄-alkylene group, wherein both definitions have the same meaning as described herein. Examples of heteroaryl-C₁₋₄-alkylene include, but are not limited to, thiophen-2-yl-methyl, thiophen-3-yl-methyl, pyrrol-1-yl-methyl, pyrrol-2-yl-methyl, pyrrol-3-yl-methyl, furan-2-yl-methyl, furan-3-yl-methyl, 2-thiophen-2-yl-ethyl, 2-thiophen-3-yl-thyl, 2-furan-2-yl-ethyl, 2-furan-3-yl-ethyl, 2-pyrrol-1-yl-ethyl, 2-pyrrol-2-yl-ethyl, 2-pyrrol-3-yl-ethyl, 3-thiophen-2-yl-propyl, 3-thiophen-3-yl-propyl, 3-furan-2-yl-propyl, 3-fuan-3-yl-propyl, 3-pyrrol-1-yl-propyl, 3-pyrrol-2-yl-propyl, 3-petrol-3-yl-propyl, and the like.

The term **"heterocyclyl"** denotes a non-aromatic carbon ring (i.e., cycloalkyl or cycloalkenyl as defined herein) wherein one, two or three ring carbons are replaced by a heteroatom selected from, but not limited to, the group consisting of O, S, N, wherein the N can be optionally substituted with H, C₁₋₄ acyl or C₁₋₄ alkyl, and ring carbon atoms optionally substituted with oxo or a thiooxo thus forming a carbonyl or thiocarbonyl group. The heterocyclic group is a 3-,4-,5-,6- or 7-member ring. Examples of a heterocyclic group include but not limited to aziridin-1-yl, aziridin-2-yl, azetidin-1-yl, azetidin-2-yl, azetidin-3-yl, piperidin-1-yl, piperidin-4-yl, morpholin-4-yl, pigerzin-1-yl, piperzin-4-yl, pyrrolidin-1-yl, pyrrolidin-3-yl, [1,3]-dioxolan-2-yl and the like.

The term **"hydroxyl"** denotes the group -**OH**.

The term **"nitro"** denotes the group -**NO₂**.

The term **"C₄₋₆ oxo-cycloalkyl"** denotes a C₄₋₇ cycloalkyl, as defined herein,

wherein one of the ring carbons is replaced with a carbonyl. Examples of C₄₋₆ oxo-cycloalkyl include, but are not limited to, 2-oxo-cyclobutyl, 3-oxo-cyclobutyl, 2-oxo-cyclopentyl, 3-oxo-cyclopentyl, 3-oxo-cyclohexyl, 2-oxo-cyclohexyl, 4-Oxo-cyclohexyl, and the like and represented by the following structures respectively: 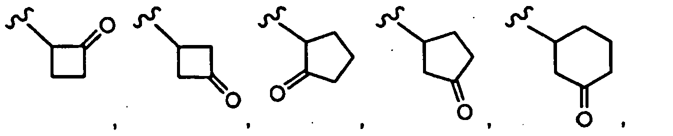 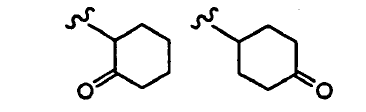 and

The term "sulfonamide" denotes the group **-SO₂NH₂.**

The term "sulfonic acid" denotes the group **-SO₃H.**

The term "thiol" denotes the group **-SH.**

The term **CODON** shall mean a grouping of three nucleotides (or equivalents to nucleotides) which generally comprise a nucleoside (adenosine (A), guanosine (G), cytidine (C), uridine (U) and thymidine (T)) coupled to a phosphate group and which, when translated, encodes an amino acid.

The term **COMPOSITION** shall mean a material comprising at least two compounds or two components; for example, and without limitation, a Pharmaceutical Composition is a Composition comprising a compound of the present invention and a pharmaceutically acceptable carrier.

The term **COMPOUND EFFICACY** shall mean a measurement of the ability of a compound to inhibit or stimulate receptor functionality, as opposed to receptor binding affinity.

The term **CONSTITUTIVELY ACTIVATED RECEPTOR** shall mean a receptor subject to constitutive receptor activation.

The term **CONSTITUTIVE RECEPTOR ACTIVATION** shall mean stabilization of a receptor in the active state by means other than binding of the receptor with its endogenous ligand or a chemical equivalent thereof.

The terms **CONTACT** or **CONTACTING** shall mean bringing the indicated moieties together, whether in an in vitro system or an in vivo system. Thus, "contacting" a **RUP25** receptor with a compound of the invention includes the administration of a compound of the present invention to an individual, for example a human, having a **RUP25** receptor, as well as, for example, introducing a compound of the invention into a sample containing a cellular or more purified preparation containing a **RUP25 receptor.**

**CORONARY HEART DISEASE** is intended herein to encompass disorders comprising a narrowing of the small blood vessels that supply blood and oxygen to the heart. CORONARY HEART DISEASE usually results from the build up of fatty material and plaque. As the coronary arteries narrow, the flow of blood to the heart can slow or stop. CORONARY HEART DISEASE can cause chest pain (stable angina), shortness of breath, heart attack, or other symptoms.

**DECREASE** is used to refer to a reduction in a measurable quantity and is used synonymously with the terms "reduce", "diminish", "lower", and "lessen".

**DIABETES** as used herein is intended to encompass the usual diagnosis of DIABETES made from any of the methods including, but not limited to, the following list: symptoms of diabetes (*e.g*., polyuria, polydipsia, polyphagia) plus casual plasma glucose levels of greater than or equal to 200 mg/dL, wherein casual plasma glucose is defined any time of the day regardless of the timing of meal or drink consumption; 8 hour fasting plasma glucose levels of less than or equal to 126 mg/dL; and plasma glucose levels of greater than or equal to 200 mg/dL 2 hours following oral administration of 75 g anhydrous glucose dissolved in water.

The phrase **DISORDERS OF LIPID METABOLISM** is intended herein to include, but not be limited to, dyslipidemia.

The term **DYSLIPIDEMIA** is intended herein to encompass disorders comprising any one of elevated level of plasma free fatty acids, elevated level of plasma cholesterol, elevated level of LDL-cholesterol, reduced level of HDL-cholesterol, and elevated level of plasma triglycerides.

The term **ENDOGENOUS** shall mean a material that a mammal naturally,produces. ENDOGENOUS in reference to, for example and not limitation, the term "receptor" shall mean that which is naturally produced by a mammal (for example, and not limitation, a human) or a virus.

In contrast, the term **NON**-**ENDOGENOUS** in this context shall mean that which is not naturally produced by a mammal (for example, and not limitation, a human) or a virus. For example, and not limitation, a receptor which is not constitutively active in its endogenous form, but when manipulated becomes constitutively active, is most preferably referred to herein as a "non-endogenous, constitutively activated receptor." Both terms can be utilized to describe both "in vivo" and "in vitro" systems. For example, and not a limitation, in a screening approach, the endogenous or non-endogenous receptor may be in reference to an in vitro screening system. As a further example and not limitation, where the genome of a mammal has been manipulated to include a non-endogenous constitutively activated receptor, screening of a candidate compound by means of an in vivo system is viable.

The phrase **IN NEED OF TREATMENT**, as used herein, refers to a judgment made by a caregiver (e.g. physician, nurse, nurse practitioner, etc. in the case of humans; veterinarian in the case of animals, including non-human mammals) that an individual or animal requires or will benefit from treatment. This judgment is made based on a variety of factors that are in the realm of a caregiver's expertise, that includes the knowledge that the individual is ill, or will be ill, as the result of a disease, condition or disorder that is treatable by the compounds of the invention. Further, the phrase "in need of treatment" also refers to the "prophylaxis" of an individual which is the judgment made by the caregiver that the individual will become ill. In this context, the compounds of the invention are used in a protective or preventive manner. Accordingly, "in need of treatment" refers to the judgment of the caregiver that the individual is already ill or will become ill and the compounds of the present invention can be used to alleviate, inhibit, ameliorate or prevent the disease, condition or disorder.

The term **INDIVIDUAL** as used herein refers to any animal, including mammals, for example, mice, rats, other rodents, rabbits, dogs, cats, swine, cattle, sheep, horses, or primates, and in one embodiment, humans.

The terms **INHIBIT** or **INHIBITING**, in relationship to the term "response" shall mean that a response is decreased or prevented in the presence of a compound as opposed to in the absence of the compound.

**INSULIN RESISTANCE** as used herein is intended to encompass the usual diagnosis of insulin resistance made by any of a number of methods, including but not restricted to: the intravenous glucose tolerance test or measurement of the fasting insulin level. It is well known that there is an excellent correlation between the height of the fasting insulin level and the degree of insulin resistance. Therefore, one could use elevated fasting insulin levels as a surrogate marker for insulin resistance for the purpose of identifying which normal glucose tolerance (NGT) individuals have insulin resistance. A diagnosis of insulin resistance can also be made using the euglycemic glucose clamp test.

The term **INVERSE AGONISTS** shall mean moieties that bind the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. In some embodiments, the baseline intracellular response is inhibited in the presence of the inverse agonist by at least 30%, in other embodiments, by at least 50%, and in still other embodiments, by at least 75%, as compared with the baseline response in the absence of the inverse agonist.

The term **LIGAND** shall mean an endogenous, naturally occurring molecule specific for an endogenous, naturally occurring receptor.

The phrase **METABOLIC-RELATED DISORDERS** is intended herein to include, but not be limited to, dyslipidemia, atherosclerosis, coronary heart disease, insulin resistance, obesity, impaired glucose tolerance, atheromatous disease, hypertension, stroke, Syndrome X, heart disease and type 2 diabetes.

As used herein, the terms **MODULATE** or **MODULATING** shall mean to refer to an increase or decrease in the amount, quality, response or effect of a particular activity, function or molecule.

The term **PHARMACEUTICAL COMPOSITION** shall mean a composition for preventing, treating or controlling a disease state or condition comprising at least one active compound, for example, a compound of the present invention including pharmaceutically acceptable salts, pharmaceutically acceptable solvates and/or hydrates thereof, and at least one pharmaceutically acceptable carrier.

The term **PHARMACEUTICALLY ACCEPTABLE CARRIER** or **EXCIPIENT** shall mean any inert substance substance used as a diluent or vehicle for a compound of the present invention.

The phrase **THERAPEUTICALLY EFFECTIVE AMOUNT** as used herein refers to the amount of active compound or pharmaceutical agent that elicits the biological or medicinal response in a tissue, system, animal, individual or human that is being sought by a researcher, veterinarian, medical doctor or other clinician, which includes one or more of the following:
(1) Preventing the disease; for example, preventing a disease, condition or disorder in an individual that may be predisposed to the disease, condition or disorder but does not yet experience or display the pathology or symptomatology of the disease,
(2) Inhibiting the disease; for example, inhibiting a disease, condition or disorder in an individual that is experiencing or displaying the pathology or symptomatology of the disease, condition or disorder (i.e., arresting further development of the pathology and/or symptomatology), and
(3) Ameliorating the disease; for example, ameliorating a disease, condition or disorder in an individual that is experiencing or displaying the pathology or symptomatology of the disease, condition or disorder (i.e., reversing the pathology and/or symptomatology).

### COMPOUNDS OF THE PRESENT INVENTION

One aspect of the present invention encompasses certain pyrazole carboxylic acid derivatives wherein both R₁ and R₄ are hydrogen atoms and can be represented by the Formula (Ia): 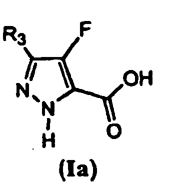 wherein:
R₃ is C₃₋₆ cycloalkyl, C₁₋₁₂ alkyl, C₁₋₁₂ haloalkyl, C₃₋₆ cycloalkyl-C₁₋₄-alkylene, aryl-C₁₋₄-alkylene or heteroaryl-C₁₋₄-alkylene, wherein said aryl-C₁₋₄-alkylene and heteroaryl-C₁₋₄-alkylene can be optionally substituted with 1 to 5 substituents selected from the group consisting of C₁₋₄ acyl, C₁₋₄ acyloxy, C₂₋₄ alkenyl, C₁₋₄ alkoxy, C₁₋₄ alkyl, C₁₋₄ alkylcarboxamide, C₂₋₄ alkynyl, C₁₋₄ alkylsulfonamide, C₁₋₄ alkylsulfinyl, C₁₋₄ alkylsulfonyl, C₁₋₄ alkylthio, C₁₋₄ alkylureyl, amino, C₁₋₄ alkylamino, C₁₋₄ dialkylamino, arylsulfonyl, carbo-C₁₋₄-alkoxy, carboxamide, carboxy, cyano, C₃₋₆ cycloalkyl, C₁₋₄ dialkylcarboxamide, C₁₋₄ dialkylsulfonamide, halogen, C₁₋₄ haloalkoxy, C₁₋₄ haloalkyl, C₁₋₄ haloalkylsulfinyl, C₁₋₄ haloalkylsulfonyl, C₁₋₄ haloalkylthio, heterocyclyl, hydroxyl, thio, nitro, C₄₋₆ oxo-cycloalkyl, sulfonamide and sulfonic acid, or
   a pharmaceutically acceptable salt, solvate or hydrate thereof.

Some embodiments of the present invention, can be represented by the Formula (Ie): 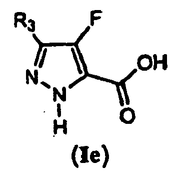 wherein R₃ in Formula (Ie) has the same meaning as described herein, *supra* and *infra*. In some embodiments, compounds of the present invention are of Formula (Ie) wherein R₃ is selected from the group consisting of cyclopropyl, -CH₃, -CH₂CH_{3,} -(CH₂)₂CH₃, and -(CH₂)₃CH₃. In further embodiments, compounds are of Formula (Ie) wherein R₃ is selected from the group consisting of -CHF₂, -CH₂F, -CH₂CF₃, -CH₂CHF₂, -CF₂CH₃, -CH₂CH₂CF₃, -CH₂CF₂CH₃, -CH₂CF₂CF₃, -CH₂CH₂CH₂CHF₂, and -CH₂CH₂CF₂CH₃. In still further embodiments, compounds are of formula (Ie) wherein R₃ is selected from the group consisting of -CF₃, -CF₂CF₃, and -(CF₂)₂CF₃. In still further embodiments, compounds are of Formula (Ie) wherein R₃ is benzyl optionally substituted with 1 to 5 substituents selected from the group consisting of C₁₋₄ alkoxy, C₁₋₄ alkyl, C₁₋₄ alkylsulfonyl, amino, C₁₋₄ alkylamino, C₁₋₄ dialkylamino, carbo C₁₋₄-alkoxy, carboxamide, carboxy, cyano, halogen, C₁₋₄ haloalkoxy, C₁₋₄ haloalkyl, and hydroxyl. In still further embodiments, compounds are of Formula (Ie) wherein R₃ is benzyl optionally substituted with 1 to 5 substituents selected from the group consisting of C₁₋₄ alkoxy, C₁₋₄ alkyl, C₁₋₄ dialkylamino, carboxamide, carboxy, cyano, halogen, and hydroxyl. In still further embodiments, compounds are of Formula (Ie) wherein R₃ is benzyl optionally substituted with 1 to 5 substituents selected from the group consisting of OCH₃, F, Cl, and Br.

in some embodiments of the present invention, R₃ is C₁₋₁₂ alkyl. In some embodiments, R₃ is selected from the group consisting of -CH₃, -CH₂CH₃, -(CH₂)₂CH₃, -CH(CH₃)₂, -(CH₂)₃CH₃, -CH₁CH(CH₃)₂, -C(CH₃)₃, -CH(CH₃)(CH₂CH₃), -(CH₂)₄CH₃, -CH₂CH₂CH(CH₃)₂, -CH(CH₃)(CH₂CH₂CH₃), -CH₂C(CH₃)₃, -CH(CH₂CH₃)₂, -CH₂CH(CH₃)CH₂CH₃, -CH(CH₃)(CH(CH₃)₂), -(CH₂)₅CH₃, -CH₂CH₂CH(CH₃)₂, -CH(CH3)(CH₂CH₂CH₂CH₃), -CH₂CH₂C(CH₃)₃, -CH(CH₂CH₃)(CH(CH₃)₂), -CH(CH₃)(C(CH₃)₃), -C(CH₃)₂(CH(CH₃)₂), -C(CH₃)(CH₂CH₃)₂, -C(CH₃)₂(CH₂CH₂CH₃), -CH(CH₃)(CH(CH₃)CH₂CH₃), -CH₂CH(CH₃)(CH(CH₃)₂), - CH₂C(CH₂CH₃)(CH₃)_{2,} -CH(CH₃)(CH₂CH(CH₃)₂), -CH(CH₂CH₃)(CH₂CH₂CH₃), -CH₂CH(CH₃)(CH₂CH₂CH₃), -CH₂CH₂CH(CH₃)(CH₂CH₃), -(CH₂)₆CH₃, -(CH₂)₇CH₃, -(CH₂)₈CH₃, -(CH₂)₉CH₃, -(CH₂)₁₀CH₃, and -(CH₂)₁₁CH₃. In some embodiments, R₃ is selected from the group consisting of -CH₃, -CH₂CH₃, -(CH₂)₂CH₃, -(CH₂)₃CH3, -(CH₂)₄CH₃, -(CH₂)₅CH₃, -(CH₂)₆CH₃, -(CH₂)₇CH₃, -(CH₂)₈CH₃, -(CH₂)₉CH₃, -(CH₂)₁₀CH₃, and -(CH₂)₁₁CH₃- In some embodiments, R₃ is selected from the group consisting of -CH₃, -CH₂CH₃, -(CH₂)₂CH₃, or -(CH₂)₃CH₃.

In some embodiments of the present invention, R₃ is C₁₋₁₂ haloalkyl. In some embodiments, R₃ is C₁₋₁₂ haloalkyl selected from the group consisting of- CHF₂, -CH₂F, -CH₂CF₃, -CH₂CHF₂, -CH₂CH₂F, -CHFCF₃, -CHFCHF₂, -CHFCH₂F, -CF₂CH₃, -CF₂CHF₂, -CF₂CH₂F, -CH₂CH₂CF₃, -CH₂CF₂CH₃, -CH₂CH₂CHF₂, -CH₂CH₂CH₂F, -CH₂CHFCF₃, -CH₂CHFCHF₂, -CH₂CHFCH₂F, -CH₂CF₂CF₃, -CH₂CF₂CHF₂, -CH₂CF₂CH₂F, -CHFCHFCF₃, -CHFCHFCHF₂, -CHFCHFCH₂F, -CHFCF₂CF₃, -CHFCF₂CHF₂, -CHF₂CH₂F, -CF₂CF₂CHF₂, -CF₂CF₂CH₂F, -CH₂CH₂CH₂CHF₂, -CH₂CH₂CF₂CH_{3,} -CH₂CH₂CH₂CF_{3,} -CH₂CH₂CF₂CF₃, -CH₂CF₂CH₂CF₃, -CF₂CH₂CH₂CF₃, -CH₂CF₂CF₂CF₃, -CF₂CH₂CF₂CF₃, -CF₂CF₂CH₂CF₃, -CF₂CH₂CH₂CH₃, -CF₂CF₂CH₂CH₃, -CF₂CH₂CF₂CH₃, -CH₂CF₂CH₂CH₃, -CH₂CF₂CF₂CH₃, -CH₂CH₂CF₂CH₃, -CH₂CH₂CH₂CH₂CF₃, -CH₂CH₂CH₂CF₂CH₃, -CH₂CH₂CF₂CH₂CH₃, -CH₂CF₂CH₂CH₂CH₃, -CF₂CH₂CH₂CH₂CH₃, -CH₂CF₂CF₂CF₂CF₃, -(CH₂)₅CF₃, -(CH₂)₄CF₂CH₃, -(CH₂)₃CF₂CH₂CH₃, -CH₂CH₂CF₂CH₂CH₂CH₃, -CH₂CF₂(CH₂)₃CH₃, -CF₂(CH₂)₄CH₃, and -CH₂(CF₂)₄CF₃- In some embodiments, R₃ is C₁₋₁₂ haloalkyl selected from the group consisting of -CHF₂, -CH₂F, -CH₂CF₃, -CH₂CHF₂, -CF₂CH₃, -CH₂CH₂CF₃, -CH₂CF₂CH₃, -CH₂CF₂CF₃, -CH₂CH₂CH₂CHF₂, and -CH₂CH₂CF₂CH₃.

In some embodiments of the present invention, R₃ is a C₁₋₁₂ fluoroalkyl selected from the group consisting of -CF₃, -CF₂CF₃, -(CF₂)₂CF₃, -CF(CF₃)₂, -(CF₂)₃CF₃, -CF₂CF(CF₃)₂, -C(CF₃)₃, -CF(CF₃)(CF₂CF₃), -(CF₂)₄CF₃, -CF₂CF₂CF(CF₃)₂, -CF(CF₃)(CF₂CF₂CF₃), -CF₂C(CF₃)₃, -CF(CF₂CF₃)₂, -CF₂CF(CF₃)CF₂CF₃, and -CF(CF₃)(CF(CF₃)₂). In some embodiments, R₃ is selected from the group consisting of -CF₃, -CF₂CF₃, -(CF₂)₂CF₃, -(CF₂)₃CF₃, and -(CF₂)₄CF₃. In some embodiments, R₃ is C₁₋₁₂ haloalkyl selected from the group consisting of -CF₃, -CF₂CF₃, and -(CF₂)₂CF₃.

In some embodiments of the present invention, R₃ is C₃₋₆ cycloalkyl. In some embodiments, R₃ is C₃₋₆ cycloalkyl selected from the group consisting of cyclopropyl, cyclobutyl, and cyclopentyl. In some embodiments, R₃ is cyclopropyl.

In some embodiments of the present invention, R₃ is C₃₋₆ cycloalkyl-C₁₋₄-alkylene optionally substituted with 1 to 5 substituents selected from the group consisting of C₂₋₄ alkenyl, C₁₋₄ alkoxy, C₁₋₄ alkyl, C₁₋₄ alkynyl, C₁₋₄ alkylsulfonyl, C₁₋₄ alkylthio, amino, C₁₋₄ alkylamino, C₁₋₄ dialkylamino, carbo-C₁₋₄-alkoxy, carboxamide, carboxy, cyano, C₃₋₆ cycloalkyl, halogen, C₁₋₄ haloalkoxy, C₁₋₄ haloalkyl, C₁₋₄ haloalkylsulfonyl, C₁₋₄ haloalkylthio, hydroxyl, thio, nitro, C₄₋₆ oxo-cycloalkyl, and sulfonamide. In some embodiments, R₃ is C₃₋₆ cycloalkyl-C₁₋₄-alkylene optionally substituted with 1 to 5 substituents selected from the group consisting of C₁₋₄ alkoxy, C₁₋₄ alkyl, C₁₋₄ alkylsulfonyl, C₁₋₄ alkylthio, carboxamide, carboxy, cyano, halogen, C₁₋₄ haloalkoxy, C₁₋₄ haloalkyl, C₁₋₄ haloalkylsulfonyl, C₁₋₄ haloalkylthio, hydroxyl, thio, nitro, and sulfonamide. In some embodiments, the C₃₋₄ cycloalkyl-C₁₋₄-alkylene is selected from the group consisting of cyclopropylmethyl, 2-cyclopropyl-ethyl, 1-cyclopropyl-ethyl, 3-cyclopropyl-propyl, 1-cyclopropyl-propyl, 1-cyclopropyl-1-methyl-ethyl, 2-cyclopropyl-1-methyl-ethyl, and 2-cyclopropyl-propyl. In some embodiments, the C₃₋₆ cycloalkyl-C₁₋₄-alkylene is cyclopropylmethyl.

In some embodiments of the present invention, R₃ is aryl-C₁₋₄-alkylene optionally substituted with 1 to 5 substituents selected from the group consisting of C₂₋₄ alkenyl, C₁₋₄ alkoxy, C₁₋₄ alkyl, C₂₋₄ alkynyl, C₁₋₄ alkylsulfonyl, C₁₋₄ alkylthio, amino, C₁₋₄ alkylamino, C₁₋₄ dialkylamino, carbo-C₁₋₄-alkoxy, carboxamide, carboxy, cyano, C₃₋₆ cycloalkyl, halogen, C₁₋₄ haloalkoxy, C₁₋₄ haloalkyl, C₁₋₄ haloalkylsulfonyl, C₁₋₄ haloalkylthio, hydroxyl, thio, nitro, C₄₋₆ oxo-cycloalkyl, and sulfonamide. In some embodiments, R₃ is aryl-C₁₋₄-alkylene optionally substituted with 1 to 5 substituents selected from the group consisting of C₁₋₄ alkoxy, C₁₋₄ alkyl, C₁₋₄ alkylsulfonyl, C₁₋₄ alkylthio, carboxamide, carboxy, cyano, halogen, C₁₋₄ haloalkoxy, C₁₋₄ haloalkyl, C₁₋₄ haloalkylsulfonyl, C₁₋₄ haloalkylthio, hydroxyl, thio, nitro, and sulfonamide. In some embodiments, the aryl-C₁₋₄-alkylene is selected from the group consisting of benzyl, phenethyl, 1-phenyl-ethyl, 3-phenyl-propyl, 1-phenyl-propyl, 1-phenyl-1-methyl-ethyl, 2-phenyl-1-methyl-ethyl, and 2-phenyl-propyl. In some embodiments, the aryl-C_{1- 4}-alkylene is benzyl.

In some embodiments of the present invention, R₃ is heteroaryl-C₁₋₄-alkylene optionally substituted with 1 to 5 substituents selected from the group consisting of C₂₋₄ alkenyl, C₁₋₄ alkoxy, C₁₋₄ alkyl, C₂₋₄ alkynyl, C₁₋₄ alkylsulfonyl, C₁₋₄ alkylthio, amino, C₁₋₄ alkylamino, C₁₋₄ dialkylamino, carbo-C₁₋₄-alkoxy, carboxamide, carboxy, cyano, C₃₋₆ cycloalkyl, halogen, C₁₋₄ haloalkoxy, C₁₋₄ haloalkyl, C₁₋₄ haloalkylsulfonyl, C₁₋₄ haloalkylthio, hydroxyl, thio, nitro, C₄₋₆ oxo-cycloalkyl, and sulfonamide. In some embodiments, R₃ is heteroaryl-C₁₋₄-alkylene optionally substituted with 1 to 5 substituents selected from the group consisting of C₁₋₄ alkoxy, C₁₋₄ alkyl, C₁₋₄ alkylsulfonyl, C₁₋₄ alkylthio, carboxamide, carboxy, cyano, halogen, C₁₋₄ haloalkoxy, C₁₋₄ haloalkyl, C₁₋₄ haloalkylsulfonyl, C₁₋₄ haloalkylthio, hydroxyl, thio, nitro, and sulfonamide. In some embodiments, the heteroaryl-C₁₋₄-alkylene is selected from the group consisting of thiophen-2-yl-methyl, thiophen-3-yl-methyl, pyrrol-1-yl-methyl, pyrrol-2-yl-methyl, pyrrol-3-yl-methyl, furan-2-yl-methyl, furan-3-yl-methyl, 2-thiophen-2-yl-ethyl, 2-thiophen-3-yl-ethyl, 2-furan-2-yl-ethyl, 2-furan-3-yl-ethyl, 2-pyrrol-1-yl-ethyl, 2-pyrrol-2-yl-ethyl, and 2-pyrrol-3-yl-ethyl. In some embodiments, the heteroaryl-C₁₋₄-alkylene is selected from the group consisting of thiophen-2-yl-methyl, thiophen-3-yl-methyl, pyrrol-1-yl-methyl, pyrrol-2-yl-methyl, pyrrol-3-yl-methyl, furan-2-yl-methyl, and furan-3-yl-methyl.

In some embodiments, R₃ is a heteroaryl-C₁₋₄-alkylene comprising a 5-atom heteroaryl ring and is represented by the following formulae in TABLE 2 below:

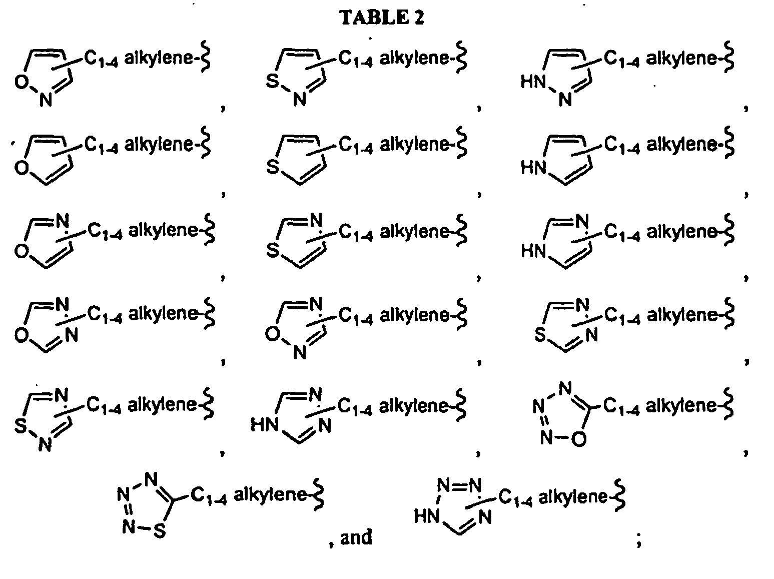

wherein the C₁₋₄-alkylene group is bonded at any available position of the 5-membered heteroaryl, for example, if the 5-membered heteroaryl is an imidazolyl ring then the C₁₋₄-alkylene group can be bonded at any one of the hollowing ring atoms, for example, one of the ring nitrogens (i.e., imidazol-1-yl group) or at one of the ring carbons (i.e., imidazol-2-yl, imidazol-4-yl or imiadazol-5-yl group). In some embodiments, the C₁₋₄-alkylene groups of TABLE 2 are -CH₂- groups. In further embodiments, the C₁₋₄-alkylene groups of TABLE 2 are -CH₂CH₂-groups.

Another aspect of the present invention includes compounds of the group consisting of: 4-fluoro-5-methyl-*2H*-pyrazole-3-carboxylic acid; 5-Fluoromethyl-4-fluoro-2*H-*pyrazole-3-carboxylic acid; 5-Difluormehyl-4-fluoro-2*H*-pyrazole-3-caboxylic acid; 5-(2-Fluoro-ethyl)-4-fluoro-*2H*-pyrazole-3-carboxylic acid; 5-(2,2-Difluoro-ethyl)-4-fluoro-*2H-*pyrazole-3-carboxylic acid; 5-(2,2,2-Trifluoro-ethyl)-4-fluoro-*2H*-pyrazole-3-carboxylic acid; 5-(1,2-Difluoro-ethyl)-4-fluoro-*2H*-pyrazole-3-carboxylic acid; 5-(1,2,2-Trifluoro-ethyl)-fluoro-*2H*-pyrazole-3-carboxylic acid; 5-(1,2,2,2-Tetrafluoro-ethyl)-4-fluoro-*2H*-pyrazole-3-carboxylic acid; 5-(1,1-Difluoro-ethyl)-4-fluoro-*2H*-pyrazole-3-carboxylic acid; 5-(1,1,2-Trifluoro-ethyl)-4-fluoro-*2H*-pyrazole-3-carboxylic acid; 5-(1,1,2,2-Tetrafluoro-ethyl)-4-fluoro-*2H*-pyrazole-3-carboxylic acid; 5-(3-Fluoro-propyl)-4-fluoro-*2H*-pyrazole-3-carboxylic acid; 5-(3,3-Difluoro-propyl)-4-fluoro-*2H*-pyrazole-3-carboxylic acid; 5-(3,3,3-Trifluoro-propyl)-4-fluoro-*2H-*pyrazole-3-carboxylic acid; 5-(2,3-Difluoro-propyl)-4-fluoro-*2H-*pyrazole-3-carboxylic acid; 5-(2,3,3-Trifluoro-propyl)-4-fluoro-*2H*-pyrazole-3-carboxylic acid; 5-(2,3,3,3-Tetrafluoro-propyl)-4-fluoro-*2H*-pyrazole-3-carboxylic acid; 5-(2,2-Difluoro-propyl)-4-fluoro-*2H*-pyrazole-3-carboxylic acid; 5-(2,2,3-Trifluoro-propyl)-4-fluoro-*2H*-pyrazole-3-carboxylic acid; 5-(2,2,3,3-Tetrafluoro-propyl)-4-fluoro-2*H* pyrazole-3-carboxylic acid; 5-(2,2,3,3,3-Pentafluoro-propyl)-4-fluoro-2*H*-pyrazole-3-carboxylic acid; 5-(1,2,3-Trifluoro-propyl)-4-fluoro-2*H*-pyrazole-3-carboxylic acid; 5-(1,2,3,3-Tetrafluoro-propyl)-4-fluoro-2*H*-pyrazole-3-carboxylic acid; 5-(1,2,3,3,3-Pentafluoro-propyl)-4-fluoro-2*H*-pyrazole-3-carboxylic acid; 5-(1,2,2,3-Tetrafluoro-propyl)-4-fluoro-2*H*-pyrazole-3-carboxylic acid; 5-(1,2,2,3,3-Pentafluoro-propyl)-4-fluoro-2*H-*pyrazole-3-carboxylic acid; 5-(1,2,2,3,3,3-Hexafluoro-propyl)-4-fluoro-2*H*-pyrazole-3-carboxylic acid; 5-(1,1,2,3-Tetrafluoro-propyl)-4-fluoro-2*H*-pyrazole-3-carboxylic acid; 5-(1,1,2,3,3-Pentafluoro-propyl)-4-fluoro-2*H*-pyrazole-3-carboxylic acid; 5-(1,1,2,3,3,3-Hexafluoro-propyl)-4-fluoro-2*H*-pyrazole-3-carboxylic acid; 5-Ethyl-4-fluoro-2*H*-pyrazole-3-carboxylic acid; 4-Fluoro-5-propyl-2*H*-pyrazole-3-carboxylic acid; 5-Butyl-4-fluoro-2*H-*pyrazole-3-carboxylic acid; 4-Fluoro-5-pentyl-2*H*-pyrazole-3-carboxylic acid; 4-Fluoro-5-hexyl-2*H*-pyrazole-3-carboxylic acid; 4-Fluoro-5-heptyl-2*H*-pyrazole-3-carboxylic acid; 4-Fluoro-5-octyl-2*H*-pyrazole-3-carboxylic acid; 4-Fluoro-5-nonyl-2*H*-pynzole-3-carboxylic acid; 5-Decyl-4-fluoro-2*H*-pyrazole-3-carboxylic acid; 4-Fluoro-5-undecyl-2*H*-pyrazole-3-carboxylic acid; 5-Dodecyl-4-fluoro-2*H*-pyrazole-3-carboxylic acid; 5-Cyclopropyl-4-fluoro-2*H*-pyrazole-3-carboxylic acid; and 5-Cyclopropylmethyl-4-fluoro-2*H*-pyrazole-3-carboxylic acid.

Another aspect of the present invention includes compounds of the group consisting of:
4-Fluoro-5-methyl-2*H*-pyrazole-3-arboxylic acid; 5-Difluoromethyl-4-fluoro-2*H* pyrazole-3-carboxylic acid; 5-(2,2-Difluoro-ethyl)-4-fluoro-2*H*-pyrazole-3-carboxylic acid; 5-(1,1-Difluoro-ethyl)-4-fluoro-2*H*-pyrazole-3-carboxylic acid; 5-(2,2-Difluoro-propyl)-4-fluoro-2*H*-pyrazole-3-carboxylic acid; 5-Ethyl-4-fluoro-2*H*-pyrazole-3-carboxylic acid; 4-Fluoro-5-propyl-2*H*-pyrazole-3-carboxylic acid; 5-Butyl-4-fluoro-2*H*-pyrazole-3-carboxylic acid; 4-Fluoro-5-pentyl-2*H*-pyrazole-3-carboxylic acid; 4-Fluoro-5-hexyl-2*H*-pyrazole-3-carboxylic acid; 4-Fluoro-5-heptyl-2*H*-pyrazole-3-carboxylic acid; 4-Fluoro-5-octyl-2*H*-pyrazole-3-carboxylic acid; 4-Fluoro-5-nonyl-2*H*-pyrazole-3-carboxylic acid; 5-Decyl-4-fluoro-2*H* pyrazole-3-carboxylic acid; 4-Fluoro-5-undecyl-2*H*-pyrazole-3-carboxylic acid; 5-Dodecyl-4-fluoro-2*H*-pyrazole-3-carboxylic acid; 5-Cyclopropyl-4-fluoro-2*H*-pyrazole-3-carboxylic acid; and 5-Cyclopropylmethyl-4-fluoro-2*H*-pyrazole-3-carboxylic acid.

### Synthetic Methods of Pyrazoles

The compounds of the present invention can be readily prepared according to a variety of synthetic regimes, all of which would be familiar to one skilled in the art. The chemical and patent literature quotes numerous procedures for the synthesis of pyrazole carboxylic acids and esters. Some of these articles include: Ashton and co-workers, J. Med. Chem. 1993, 36, 3595-3605; Seki and co-workers, Chem. Pharm. Bull., 1984, 32, 1568; and Wiley and Hexner, Org. Syn Coll IV, 1963, 351.
For the purposes of describing the syntheses below, the following compounds of Formula (I) are defined: 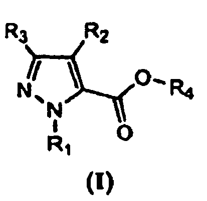 wherein:
R₁ is H, C₁₋₁₂ alkyl or C₁₋₁₂ haloalkyl;
R₂ is H, halogen, C₁₋₁₂ alkyl or C₁₋₁₂ haloalkyl;
R₃ is C₃₋₆ cycloalkyl, C₁₋₁₂ alkyl, C₁₋₁₂ haloalkyl, C₁₋₆ cycloalkyl-C₁₋₄-alkylene, aryl-C₁₋₄-alkylene or heteroaryl-C₁₋₄-alkylene, wherein said aryl-C₁₋₄-alkylene and heteroaryl-C₁₋₄-alkylene can be optionally substituted with 1 to 5 substituents selected from the group consisting of C₁₋₄ acyl, C₁₋₄ acyloxy, C₂₋₄ alkyl, C₁₋₄ alkoxy, C₁₋₄ alkyl, C₁₋₄ alkylcarboxamide, C₂₋₄ alkynyl, C₁₋₄ alkylsulfonamide, C₁₋₄ alkylsulfinyl, C₁₋₄ alkylsulfonyl, C₁₋₄ alkylthio, C₁₋₄ alkylureyl, amino, C₁₋₄ alkylamino, C₁₋₄ dialkylamino, arylsulfonyl, carbo-C₁₋₄-alkoxy, carboxamide, carboxy, cyano, C₃₋₄ cycloalkyl, C₁₋₄ dialkylcarboxamide, C₁₋₄ dialkylsulfonamide, halogen, C₁₋₄ haloalkoxy, C₁₋₄ haloalkyl, C₁₋₄ haloalkylsulfinyl, C₁₋₄ haloalkylsulfonyl, C₁₋₄ haloalkylthio, heterocyclyl, hydroxyl, thio, nitro, C₄₋₆ OXO-cycloalkyl, sulfonamide and sulfonic acid; and
R₄ is H or C₁₋₁₂ alkyl; or
a pharmaceutically acceptable salt, solvate or hydrate thereof.

The methods described below may be used for the preparation of compounds of the invention.

One method that may be used to prepare compounds of Formula (**I**) utilizes intermediates of Formula (**A**) as illustrated in Reaction Scheme (**1**) below:

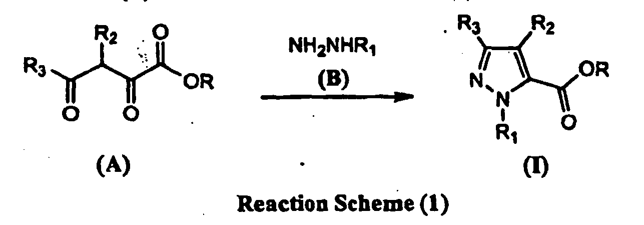

Compounds of Formula **(I)** may be prepared by treating the 2,4-diketo ester or acid [R = alkyl or H respectively, **(A)**] with hydrazine **(B)** under various conditions. For example, the solvent may optionally be present or absent. In the instance that the solvent is absent then the hydrazine **(B)** serves both as a reactant and as the solvent. Typically, under these conditions hydrazine would be present in molar excess. In the instance when the solvent is present, the solvent may be a polar solvent and is generally a C₁-C₆ alcohol. Some typical solvents may be selected from, but not limited to, the group consisting of methanol, ethanol, butanol, pentanol, hexanol, 2-methoxyethanol, 1-propanol and 2-propanol. In some instances it may be beneficial to include the presence of an acid. Some representative examples of acids may be selected from the group consisting of hydrochloric acid, hydrobromic acid, acetic acid and trifluoroacetic acid. The reaction temperature generally ranges from about 20°C to about 160°C, and for convenience, the reaction temperature is typically the reflux temperature of the reaction mixture.

The 2,4-diketo esters or acids **(A)** may be readily available or may be obtained by methods known in the art, Seki and co-workers, Chem. Pharm. Bull., 1984, 32, 1568. Likewise, the hydrazines of Formula **(B)** may be readily available from commercial sources or may be obtained by methods know in the art.

One particular feature of 2,4-diketo esters or acids **(A)** is that a diverse number of R₂ groups may be introduced by a variety of methods known in the art, such as, alkylation, as shown in Reaction Scheme **(2)** below: 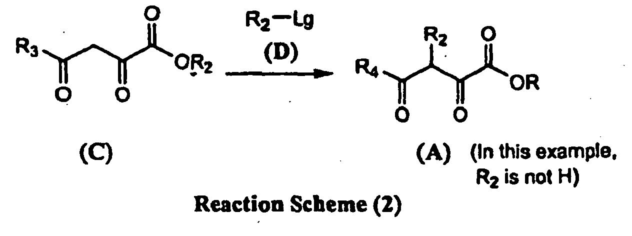 The alkylation step as shown in Reaction Scheme **(2)** is similar to and in some instances identical to that described in the preparation of intermediate **(F)**, *infra.*

Utilizing a similar starting material as in Reaction Scheme **(1)** an alternative method may be used to prepare compounds of Formula **(I)** as illustrated in Reaction Scheme **(3)** below:

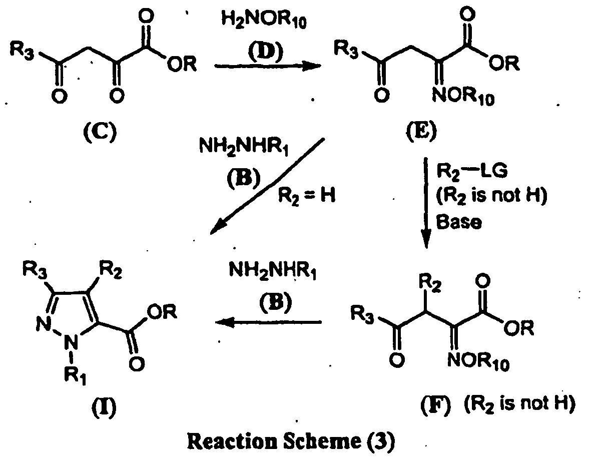

Compounds of Formula **(I)** may be prepared by treating 2,4-diketo ester or acid **(C),** in some embodiments the ester, with an alkoxyamine of Formula **(D)** where R₁₀ is C₁-C₆ alkyl, leading to 2-(methoxyimino) intermediate of Formula **(E).** Typically, the alkoxyamine is methoxyamine (i.e., O-methyl hydroxylamine) wherein R₁₀ is methyl. This step is typically conducted in the presence of a drying agent to concomitantly remove the water formed during the process; examples of a drying agent that may be used include molecular sieves, magnesium sulfate and the like. In the subsequent step, the intermediate of Formula **(E)** may be functionalized with R₂ utilizing methods known in the art. One example may use R₂-LG, wherein LG is a leaving group, such as, iodo, bromo, mesylate and the like, in the presence of a base and a polar solvent Typical bases may be selected from, potassium carbonate, sodium carbonate, sodium hydroxide, potassium hydroxide, lithium hydroxide, LDA, sodium methoxide, sodium ethoxide and the like; and the polar solvent may be dimethylformamide, dimethylsulfoxide, THF and the like. A depicted in Reaction Scheme **(3),** intermediate **(E)** may be converted to a compound of Formula **(I)** wherein R₂ is H using hydrazine **(B).** This step may be performed under heating conditions in an alcoholic solvent as described above in Reaction Scheme **(1).** Optionally, an acid may be present, such as HCl Similarly, in the example where R₂ is not **H,** intermediate **(F)** may be treated with hydrazine **(B)** in a manner as described above to provide compounds of Formula **(I)** where R₂ is a group other than H.

It is understood that in reference to Reaction Scheme **(3),** a compound of Formula **(C)** may be functionalized with R₂ as described above prior to treating with alkoxyamine **(D)** to give the same intermediate **(F).** Absent any chemical reason that would be known in the art, the order of the steps may be changed and may be more a matter of convenience than necessity [i.e., **(C)** to **(E)** to **(F);** or **(C)** to **(A)** to **(F)].**

In addition, compounds of the invention may be prepared using transition metal carbene complexes as illustrated in Reaction Scheme **(4)** below:

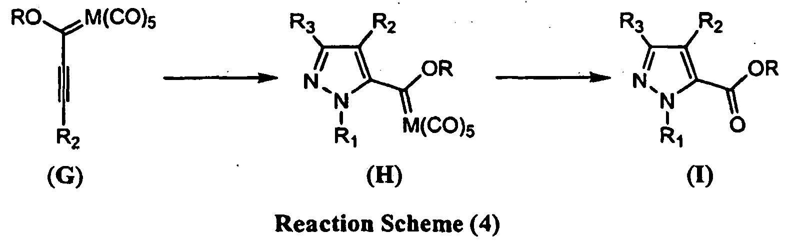

Various methods may be used in the preparation of carbene complexes, such as, those methods reviewed by Dotz and co-workers, Transition Metal Carbeen Complexes, VCH, Deerfield Beach FL, 1983 and by Brown, Prog. Inorg. Chem. 1980, 27, 1. The intermediate **(G),** where M can be chromium or tungsten, may be converted to a pyrazole carbene complex **(H)** by treatment with diazomethane or a derivative of diazomethane, such as trimethylsilyldiazomethane, and the like. Typically, intermediate **(H)** need not be isolated but may be directly oxidized to pyrazoles of Formula (**I**). Acceptable oxidants include, for example, ceric ammonium nitrate (i.e., CAN), and the like. One advantage of this method is that the cycloaddition step is typically highly regioselective. Generally, this particular method may be most appropriate for when R₃ is H.

In addition, compounds of the invention may be prepared by another method through the use of trihaloacetyl enol ethers as shown in Reaction Scheme (5) below:

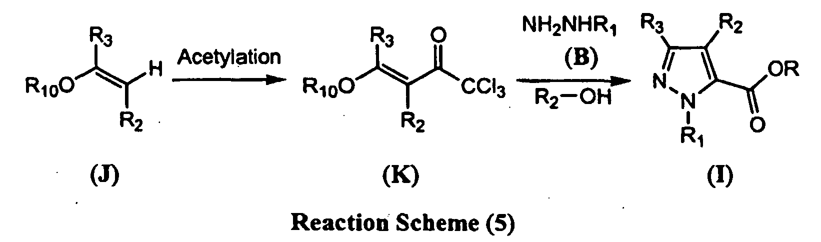

The enol ethers **(J)** are available or may be prepared by various methods known in the art. Intermediate **(K)** may be prepared by treating enol ether **(J)** with an acetylating agent under basic conditions at reduced temperatures. A variety of acetylating agents may be utilized, such as, trichloroacetyl chloride, trichloroacetic anhydride, trifluoroacetyl chloride, trifluoroacetic anhydride, and the like. Typically, the solvent is pyridine and the reaction temperature is at 25°C or below; however, typically is 0°C or below. The trihaloacetyl enol ether **(K)** may be treated with hydrazine **(B)** in a polar solvent and heated to reflux to give compound of Formula **(I).** Typically, the polar solvent is a C₁-C₆ alcohol as described above.

Certain examples of Formula **(I)** when R₁ is H may be N-alkylated by a variety of methods known in art. Alkylating reagents may be of the general formula R₁-LG where R₁ and LG have the meaning as described herein *supra.* For example, methylation may be realized using reagents such as dimethylsulfate, methyl iodide, and the like. Other R₁ groups may be used through the use of similar reagents, such as ethyl iodide, ethyl bromide, propyl iodide, isopropyl bromide, trifluoromethyl iodide, 2,2,2-trifluoroethyl-1-iodide, and the like. Generally a base is used to facilitate the N-alkylation.

Esters of the compounds shown, *supra,* wherein R = alkyl, may be readily converted to the corresponding carboxylic acids of Formula **(I),** (i.e., in example cited above R = H) by methods known in the art, such as alkaline hydrolysis using LiOH, NaOH, KOH, and the like. Another method for the conversion of an ester to a carboxylic acid of Formula **(I)** is through the use of acid hydrolysis, such as aqueous HCl and the like. Generally, the solvent is an aqueous mixture with a polar solvent as described above.

The present invention also encompasses diastereomers as well as optical isomers, e.g. mixtures of enantiomers including racemic mixtures, as well as individual enantiomers and diastereomers, which arise as a consequence of structural asymmetry in certain compounds of the present invention. Separation of the individual isomers or selective synthesis of the individual isomers is accomplished by application of various methods which are well known to practitioners in the art.

The various organic group transformations utilized herein may be performed by a number of procedures other than those described above. References for other synthetic procedures that may be utililized for the preparation of intermediates or compounds disclosed herein may be found in, for example, Smith, M. B.; and March, J., Advanced Organic Chgermistry, 5th Edition, Wiley-Interscience (2001); Larock, R.C., Comprehensive Organic Transformations, A Guide to Functional Group Preparations, 2nd Edition, VCH Publishers, Inc. (1999), or Wuts, P. G. M.; Greene, T. W.; Protective Groups in Organic Synthesis, 3rd Edition, John Wiley and Sons, (1999).

Compounds disclosed herein may exist in various tautomeric forms. For example, when R₁ is a hydrogen atom in hydrazine **(B)** then the resulting compounds formed in the process as described in the Reaction Schemes, *supra,* would be represented by Formula **(II)** as shown below: 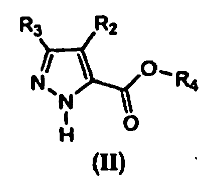 Although Formula **(II)** depicts only one of the possible tautomers, it is well understood and appreciated that pyrazole compounds may exist in various tautomeric forms, such as those shown below: 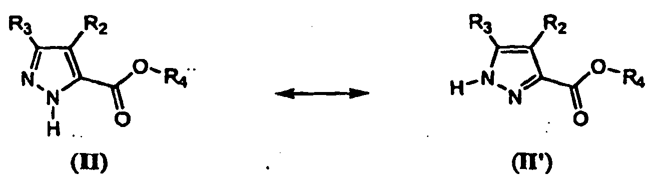 Therefore, for convenience, tautomers **(II)** and **(II')** are presented herein by one single formulae, such as, Formulae **(I), (Ia), (Ic), (Ie)** and the like, for when R₁ is **H.** It is understood that all tautomers of the compounds disclosed herein are within the scope of the invention. Further, based on the chemical formula there can be at least two different names for the same compound, such chemical naming rules are known to those skilled in the art. For example, the compound shown below represents one possible tautomer and based on the chemical representation is appropriately named 5-Ethyl-4-fluoro-2H-pyrazole-3-carboxylic acid. 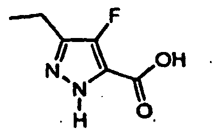 However, as discussed *supra,* this compound can also have another tautomeric form as shown below and based on this chemical representation is appropriately named 5-Ethyl-4-fluoro-1H-pyrazole-3-carboxylic acid. 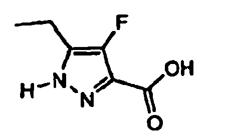 Accordingly, there are at least two possible correct names for the same compound based on the chemical formula. It is understood that both of these names, as well as others based on the priority of the attached groups, are correct and fully embraced by the present invention.

Representative examples are shown in Table 3.

**TABLE 3**

| **R₁** | **R₂** | **R₃** |
|---|---|---|
| -H | -F | -CH₃ |
| -H | -F | -CH₂F |
| -H | -F | -CHF₂ |
| -H | -F | -CH₂CH₂F |
| -H | -F | -CH₂CHF₂ |
| -H | -F | -CH₂CF₃ |
| -H | -F | -CHFCH₂F |
| -H | -F | -CHFCHF₂ |
| -H | -F | -CHFCF₃ |
| -H | -F | -CF₂CH₃ |
| -H | -F | -CF₂CH₂F |
| -H | -F | -CF₂CHF₂ |
| -H | -F | -CH₂CH₂CH₂F |
| -H | -F | -CH₂CH₂CHF₂ |
| -H | -F | -CH₂CH₂CF₃ |
| -H | -F | -CH₂CHFCH₂F |
| -H | -F | -CH₂CHFCHF₂ |
| -H | -F | -CH₂CHFCF₃ |
| -H | -F | -CH₂CF₂CH₃ |
| -H | -F | -CH₂CF₂CH₂F |
| -H | -F | -CH₂CF₂CHF₂ |
| -H | -F | -CH₂CF₂CF₃ |
| -H | -F | -CHFCHFCH₂F |
| -H | -F | -CHFCHFCHF₂ |
| -H | -F | -CHFCHFCF₃ |
| -H | -F | -CHFCF₂CH₂F |
| -H | -F | -CHFCF₂CHF₂ |
| -H | -F | -CHFCF₂CF₃ |
| -H | -F | -CF₂CHFCH₂F |
| -H | -F | -CF₂CHFCHF₂ |
| -H | -F | -CF₂CHFCF₃ |
| -H | -F | -CH₂CH₃ |
| -H | -F | -CH₂CH₂CH₃ |
| -H | -F | -CH₂(CH₂)₂CH₃ |
| -H | -F | -CH₂(CH₂)₃CH₃ |
| -H | -F | -CH₂₍CH₂)₄CH₃- |
| -H | -F | -CH₂(CH₂)₅CH₃ |
| -H | -F | -CH₂(CH₂)₈CH₃ |
| -H | -F | -CH₂(CH₂)₇CH₃ |
| -H | -F | -CH₂(CH₂)₈CH₃ |
| -H | -F | -CH₂(CH₂)CH₃ |
| -H | -F | -CH₂(CH₂)₁₀CH₃ |
| -H | -F | -C-C₃H₈ |
| -H | -F | -CH₂C-C₃H₈ |
| -H | -F | -CH₂-(3-F-phenyl) |
| -H | -F | -CH₂-(3-Br-phenyl) |
| -H | -F | -CH₂-(4-Br-phenyl) |
| -H | -F | -CH₂CH₂-(4-OCH₃-phenyl) |

The term c-C₃H₆, as defined herein, refers to the cyclopropyl group.

### PHARMACEUTICAL COMPOSITIONS

One aspect of the present invention encompasses pharmaceutical compositions comprising a pharmaceutically acceptable carrier or excipient and a compound as disclosed herein. Some embodiments of the present invention pertain to pharmaceutical compositions comprising a pharmaceutically acceptable carrier or excipient and a compound of Formula **(Ia).**

Some embodiments of the present invention include methods of producing a pharmaceutical composition comprising admixing a compound according to any of the compound embodiments disclosed herein and a pharmaceutically acceptable carrier. Some embodiments of the present invention pertain to methods of producing a pharmaceutical composition comprising admixing a compound of Formula **(Ia)** or any of the compound embodiments disclosed herein and a pharmaceutically acceptable carrier.

Compounds of the present invention or pharmaceutically acceptable salt and/or a solvate and/or a hydrate or physiologically functional derivative thereof can be used as an active ingredient in pharmaceutical compositions. The term "active ingredient" is defined in the context of a "pharmaceutical composition" and shall mean a component of a pharmaceutical composition that provides the primary pharmacological effect, as opposed to an "inactive ingredient" which would generally be recognized as providing no pharmaceutical benefit. An example of "active ingredient" is a compound of the present invention, another example includes an agent used in combination therapy, such as a sulfonylurea, meglitinide, biguanide, and like agents as discussed herein.

Formulations may be prepared by any suitable method, typically by uniformly mixing the active compound(s) with liquids or finely divided solid carriers, or both, in the required proportions, and then, if necessary, forming the resulting mixture into a desired shape.

Conventional excipients, such as binding agents, fillers, acceptable wetting agents, tabletting lubricants, and disintegrants may be used in tablets and capsules for oral administration. Liquid preparations for oral administration may be in the form of solutions, emulsions, aqueous or oily suspensions, and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives, and flavorings and colorants may be added to the liquid preparations. Parenteral dosage forms may be prepared by dissolving the compound of the invention in a suitable liquid vehicle and filter sterilizing the solution before filling and sealing an appropriate vial or ampoule. These are just a few examples of the many appropriate methods well known in the art for preparing dosage forms.

A compound of the present invention can be formulated into pharmaceutical compositions using techniques well known to those in the art. Suitable pharmaceutically-acceptable carriers, outside those mentioned herein, are known in the art; for example, see Remington, The Science and Practice of Pharmacy, 20th Edition, 2000, Lippincott Williams & Wilkins, (Editors: Gennaro, A. R., et al.).

While it is possible that, for use in the treatment of a metabolic-related disorder, compounds of the invention may, in an alternative use, be administered as a raw or pure chemical, it is preferable however, to administer the compounds or active ingredients as a pharmaceutical formulation or composition further comprising a pharmaceutically acceptable carrier.

The present invention further provides pharmaceutical formulations comprising a compound of the invention or a pharmaceutically acceptable salt or derivative thereof together with a pharmaceutically acceptable carriers thereof. The carrier(s) must be "acceptable" in the sense of being compatible with the other ingredients of the formulation and not overly deleterious to the recipient thereof.

"Pharmaceutical compositions" or "Formulations" include those compositions suitable for oral, rectal, nasal, topical (including buccal and sub-lingual), vaginal or parenteral (including intramuscular, sub-cutaneous and intravenous) administration or in a form suitable for administration by inhalation, insufflation or by a transdermal patch. Transdermal patches dispense a drug at a controlled rate by presenting the drug for absorption in an efficient manner with a minimum of degradation of the drug. Typically, transdermal patches comprise an impermeable backing layer, a single pressure sensitive adhesive and a removable protective layer with a release liner. One of ordinary skill in the art will understand and appreciate the techniques appropriate for manufacturing a desired efficacious transdermal patch based upon the needs of the artisan.

The compounds of the invention, together with a pharmaceutically acceptable carrier, adjuvant, or diluent, may be placed into the form of pharmaceutical formulations and unit dosages thereof, and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition, in some embodiments, is made in the form of a dosage unit containing a particular amount of the active ingredient, for example, a compound disclosed herein. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

The dose when using the compounds of the present invention can vary within wide limits, and as is customary and is known to the physician, it is to be tailored to the individual conditions in each individual case. It depends, for example, on the nature and severity of the illness to be treated, on the condition of the individual, on the compound employed or on whether an acute or chronic disease state is treated or prophylaxis is conducted or on whether further active compounds are administered in addition to the compounds of the present invention. Representative doses of the present invention include, but not limited to, about 0.001 mg to about 5000 mg, about 0.001 to about 2500 mg, about 0.001 to about 1000 mg, about 0.001 to about 500 mg, about 0.001 mg to about 250 mg, about 0.001 mg to 100 mg, about 0.001 mg to about 50 mg, and about 0.001 mg to about 25 mg. Depending on the individual and as deemed appropriate from the individual's physician or care-giver it may be necessary to deviate upward or downward from the doses described herein. It will be obvious to those skilled in the art that the dosage forms may comprise as the active component, either a compound of the invention or a pharmaceutically acceptable salt of a compound of the invention; or pharmaceutically acceptable solvate or hydrate thereof.

The amount of active ingredient, or an active salt or derivative thereof, required for use in treatment will vary not only with the particular salt selected but also with the route of administration, the nature of the condition being treated and the age and condition of the individual and will ultimately be at the discretion of the attendant physician or clinician. In general, one skilled in the art understands how to extrapolate in vivo data obtained in a model system, typically an animal model, to another, such as a human. Typically, animal models include, but are not limited to, the rodents diabetes models as described in Example 1, *infra,* or the mouse artherosclerosis model as described in Example 2, *infra.* Other animal models are known in the art, for example, those reported by Reed and Scribner in Diabetes, Obesity and Metabolism,1,1999, 75-86. In some circumstances, these extrapolations may merely be based on the weight of the animal in the respective model in comparison to another, such as a mammal, for example, a human, however, more often, these extrapolations are not simply based on weights, but rather incorporate a variety of factors. Representative factors include, but not limited to, the type, age, weight, sex, diet and medical condition of the individual, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated or prophylaxis is conducted or on whether further active compounds are administered in addition to the compounds of the present invention and as part of a drug combination. The dosage regimen for treating a disease condition with the compounds and/or compositions of this invention is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods of this invention.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations. The daily dose can be divided, especially when relatively large amounts are administered as deemed appropriate, into several, for example 2, 3 or 4, part administrations. If appropriate, depending on individual behavior, it may be necessary to deviate upward or downward from the daily dose indicated.

For preparing pharmaceutical compositions from the compounds of the present invention, the selection of a suitable pharmaceutically acceptable carrier can be either solid, liquid or a mixture of both. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted to the desired shape and size.

The powders and tablets may contain varying percentage amounts of the active compound. A representative amount in a powder or tablet may contain from 0.5 to about 90 percent of the active compound; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

For topical administration to the epidermis the compounds according to the invention may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the individual administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided a pressurized pack with a suitable propellant. If the compounds of the Formula (Ia) or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds of the Formula (Ia) as an aerosol can be prepared by processes well-known to the person skilled in the art. For their preparation, for example, solutions or dispersions of the compounds of the present invention in water, water/alcohol mixtures or suitable saline solutions can be employed using customary additives, for example benzyl alcohol or other suitable preservatives, absorption enhancers for increasing the bioavailability, solubilizers, dispersants and others, and, if appropriate, customary propellants, for example include carbon dioxide, CFCs such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

The pharmaceutical preparations are, in some embodiments, in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

In some embodiments of the present invention, tablets or capsules are used for oral administration and liquids for intravenous administration.

The compounds according to the invention may optionally exist as pharmaceutically acceptable salts including pharmaceutically acceptable acid addition salts prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfuric, tartaric, oxalic, p-toluenesulfonic and the like, such as those pharmaceutically acceptable salts listed in Journal of Pharmaceutical Science, 66,2 (1977).

The acid addition salts may be obtained as the direct products of compound synthesis. In the alternative, the free base may be dissolved in a suitable solvent containing the appropriate acid, and the salt isolated by evaporating the solvent or otherwise separating the salt and solvent. The compounds of this invention may form solvates with standard low molecular weight solvents using methods known to the skilled artisan.

Compounds of the present invention can be converted to "pro-drugs." The term "pro-drugs" refers to compounds that have been modified with specific chemical groups known in the art and when administered into an individual these groups undergo biotransformation to give the parent compound. Pro-drugs can thus be viewed as compounds of the invention containing one or more specialized non-toxic protective groups used in a transient manner to alter or to eliminate a property of the compound. In one general aspect, the "pro-drug" approach is utilized to facilitate oral absorption. A thorough discussion is provided in T. Higuchi and V. Stella, "Pro-drugs as Novel Delivery Systems," Vol.14 of the A.C.S. Symposium Series; and in Bioreversible Carriers in Drug Design, ed. Edward B. Roche, American Pharmaceutical Association and Pergamon Press, 1987.

It is noted that when the **RUP25** receptor modulators are utilized as active ingredients in a pharmaceutical composition, these are not intended for use only in humans, but in other non-human mammals as well. Indeed, recent advances in the area of animal health-care suggest that consideration be given for the use of active agents, such as **RUP25** receptor modulators, for the treatment of metabolic-related disorders in domestic animals (*e.g*., cats and dogs), and **RUP25** receptor modulators in other domestic animals where no disease or disorder is evident (*e.g*., food-oriented animals such as cows, chicken, fish, etc.). Those of ordinary skill in the art are readily credited with understanding the utility of such compounds in such settings.

### INDICATIONS, METHODS AND USE OF COMPOUNDS

Described herein are methods of modulating a **RUP25** receptor comprising contacting the receptor with an effective amount of a compound of the present invention. In some embodiments, the compound is an agonist of the **RUP25** receptor. In some embodiments, the compound is a partial agonist of the **RUP25** receptor. In some embodiments, the compound is a full agonist of the **RUP2S** receptor.

Described herein are methods of modulating a **RUP25** receptor in an individual comprising contacting the receptor with an effective amount of a compound of the present invention. In some embodiments, the modulation treats a metabolic-related disorder.

Described herein are methods of modulating **RUP25** receptor function in an *in vitro* system, such as a cell or isolated tissue, or in an in vivo system, such as an intact tissue or individual, comprising contacting said cell, tissue or individual with an effective amount of a compound or pharmaceutical composition as described herein. In some embodiments, the **RUP25** receptor function is associated with a metabolic-related disorder.

Described herein are methods of treatment of a metabolic-related disorder comprising administering to an individual in need of such treatment a therapeutically effective amount of a compound according to any of the embodiments disclosed herein or pharmaceutical composition according to any of the embodiments disclosed herein. In some embodiments, the pharmaceutical composition is a compound, as described herein, a pharmaceutically acceptable carrier, and an agent selected from the group consisting of α-glucosidase inhibitor, aldose reductase inhibitor, biguanide, HMG-CoA reductase inhibitor, squalene synthesis inhibitor, fibrate, LDL catabolism enhancer, angiotensin converting enzyme inhibitor, insulin secretion enhancer and thiazolidinedione.

In some embodiments of the present invention, the individual is a mammal. In some embodiments, the mammal is a human.

Another aspect of the present invention pertains to a compound, as described herein, for use in a method of treatment of the human or animal body by therapy.

Another aspect of the present invention pertains to a compound, as described herein, for use in a method of treatment of a metabolic-related disorder of the human or animal body by therapy.

In some embodiments, the metabolic-related disorder is selected from the group consisting of dyslipidemia, atherosclerosis, coronary heart disease, insulin resistance, obesity, impaired glucose tolerance, atheromatous disease, hypertension, stroke, Syndrome X, heart disease and type 2 diabetes. In some embodiments, the metabolic-related disorder is selected from the group consisting of dyslipidemia, atherosclerosis, coronary heart disease, insulin resistance and type 2 diabetes.

Another aspect of the present invention pertains to the use of a compound as described herein, for the manufacture of a medicament for use in the treatment of a metabolic-related disorder.

Another aspect of the prevent invention pertains to the use of a compound of Formula **(Ia)** for the manufacture of a medicament for use in the treatment of a metabolic-related disorder.

In some embodiments, the present invention pertains to the use of a compound as described herein, for the manufacture of a medicament for use in the treatment of a metabolic-related disorder further comprises an agent selected from the group consisting of α-glucosidase inhibitor, aldose reductase inhibitor, biguanide, HMG-CoA reductase inhibitor, squalene synthesis inhibitor, fibrate, LDL catabolism enhancer, angiotensin converting enzyme inhibitor, insulin secretion enhancer and thiazolidinedione.

In addition to the foregoing beneficial methods and uses for the compounds of the present invention, compounds of the invention are useful in the treatment of additional diseases. It is also well-established that metabolic diseases exert a negative influence on other physiological systems. Thus, there is often the codevelopment of multiple disease states or secondary diseases with the primary disease (e.g. kidney disease, peripheral neuropathy). Thus, treatment of the primary condition will in turn be beneficial to such interconnected disease states. Without limitation, these include the following.

### COMBINATION THERAPY

One aspect of the invention encompasses pharmaceutical compositions, as described herein, further comprising an agent (also referred to a pharmaceutical agent herein) selected from the group consisting of α-glucosidase inhibitor, aldose reductase inhibitor, biguanide, HMG-CoA reductase inhibitor, squalene synthesis inhibitor, fibrate, LDL catabolism enhancer, angiotensin converting enzyme inhibitor, insulin secretion enhancer and thiazolidinedione.

In some embodiments of the invention the pharmaceutical composition comprises an α-glucosidase inhibitor. In some embodiments the α-glucosidase inhibitor is acarbose, voglibose or miglitol. In some embodiments the α-glucosidase inhibitor is voglibose.

In some embodiments of the invention the pharmaceutical composition comprises an aldose reductase inhibitor. In some embodiments the aldose reductase inhibitor is tolurestat; epalrestat; imirestat; zenarestat; zopolrestat; or sorbinil.

In some embodiments of the invention the pharmaceutical composition comprises a biguanide. In some embodiments the biguanide is phenformin, metformin or buformin. In some embodiments the biguanide is metformin.

In some embodiments of the invention the pharmaceutical composition comprises a HMG-CoA reductase inhibitor. In some embodiments the HMG-CoA reductase inhibitor is rosuvastatin, pravastatin, simvastatin, lovastatin, atorvastatin, fluvastatin or cerivastatin.

In some embodiments of the invention the pharmaceutical composition comprises a fibrate. In some embodiments the fibrate is bezafibrate, beclobrate, binifibrate, ciplofibrate, clinofibrate, clofibrate, clofibric acid, etofibrate, fenofibrate, gemfibrozil, nicofibrate, pirifibrate, ronifibrate, simfibrate, or theofibrate.

In some embodiments of the invention the pharmaceutical composition comprises an angiotensin converting enzyme inhibitor. In some embodiments the angiotensin converting enzyme inhibitor is captopril, enalapril, alacepril, delapril; ramipril, lisinopril, imidapril, benazepril, ceronapril, cilazapril, enalaprilat, fosinopril, moveltopril, perindopril, quinapril, spirapril, temocapril or trandolapril.

In some embodiments of the invention the pharmaceutical composition comprises an insulin secretion enhancer. In some embodiments the insulin secretion enhancer is tolbutamide; chlorpropamide; tolazamide; acetohexamide; glycopyramide; glibenclamide; gliclazide; 1-butyl-3-metanilylurea; carbutamide; glibonuride; glipizide; gliquidone; glisoxepid; glybuthiazole; glibuzole; glyhexamide; glymidine; glypinamide; phenbutamide; tolcyclamide, glimepiride, nateglinide, or mitiglinide.

In some embodiments of the invention the pharmaceutical composition comprises a thiazolidinedione. In some embodiments the thiazolidinedione is rosiglitazone or pioglitazone. In some embodiments the thiazolidinedione is rosiglitazone.

It will be understood that the combination-therapy of the compounds of the present invention with other pharmaceutical agents is not limited to those listed herein, *supra* or *infra,* but includes in principle any combination with any pharmaceutical agent or pharmaceutical composition useful for the treatment of a disease, condition, disorder or symptom linked to a metabolic-related disorder.

Described herein are methods of treatment of a disease, condition, disorder or symptom thereof as described herein, comprising administering to an individual in need of such treatment a therapeutically effective amount or dose of a compound of the present invention in combination with at least one pharmaceutical agent selected from the group consisting of: sulfonylureas, meglitinides, biguanides, α-glucosidase inhibitors, peroxisome proliferators-activated receptor-γ (i.e., PPAR-γ) agonists, insulin, insulin analogues, HMG-CoA reductase inhibitors, cholesterol-lowering drugs (for example, fibrates that include: fenofibrate, bezafibrate, gemfibrozil, clofibrate and the like; bile acid sequestrants which include: cholestyramine, colestipol and the like; and niacin), antiplatelet agents (for example, aspirin and adenosine diphosphate receptor antagonists that include: clopidogrel, ticlopidine and the like), angiotensin-converting enzyme inhibitors, angiotensin II receptor antagonists and adiponectin. In some embodiments, methods of the present invention include compounds of the present invention and the pharmaceutical agents are administered separately. In further embodiments, compounds of the present invention and the pharmaceutical agents are administered together.

Suitable pharmaceutical agents that can be used in conjunction with compounds of the present invention include the α-glucosidase inhibitors. The α-glucosidase inhibitors competitively inhibit digestive enzymes such as α-amylase, maltase, α-extrinase, sucrase, etc. in the pancreas and or small intestine. The reversible inhibition by α-glucosidase inhibitors retard, diminish or otherwise reduce blood glucose levels by delaying the digestion of starch and sugars. Examples of α-glucosidase inhibitors include acarbose, N-(1,3-dihydroxy-2-propyl)valiolamine (generic name; voglibose), miglitol, and α-glucosidase inhibitors known in the art.

Suitable pharmaceutical agents that can be used in conjunction with compounds of the present invention include aldose reductase inhibitors. Aldose reductase inhibitors inhibit the first-stage rate-limiting enzyme in the polyol pathway that prevent or arrest diabetic complications. In the hyperglycemic state of diabetes, the utilization of glucose in the polyol pathway is increased and the excess sorbitol accumulated intracellularly as a consequence acts as a tissue toxin and hence evokes the onset of complications such as diabetic neuropathy, retinopathy, and nephropathy. Examples of the aldose reductase inhibitors include tolurestat; epalrestat; 3,4-dihydro-2,8-diisopropyl-3-thioxo-2*H*-1,4-benzoxazine-4-acetic acid; 2,7-difluorospiro(9*H*-fluorene-9,4'-imidazolidine)-2',5'-dione (generic name: imirestat); 3-[(4-bromo-2-flurophenyl)methy]-7-chloro-3,4-dihydro-2,4-dioxo-1(2*H*)-quinazoline acetic acid (generic name: zenarestat); 6-fluoro-2,3-dihydro-2',5'-dioxo-spiro[4*H*-1-benzopyran-4,4'-imidazolidine]-2-carboxamide (SNK-860); zopolrestat; sorbinil; and 1-[(3-bromo-2-benzofuranyl)sulfonyl]-2,4-imidazolidinedione (M-16209), and aldose reductase inhibitors known in the art.

Suitable pharmaceutical agents that can be used in conjunction with compounds of the present invention include the biguanides. The biguanides represent a class of drugs that stimulate anaerobic glycolysis, increase the sensitivity to insulin in the peripheral tissues, inhibit glucose absorption from the intestine, suppress of hepatic gluconeogenesis, and inhibit fatty acid oxidation. Examples of biguanides include phenformin, metformin, buformin, and biguanides known in the art.

Suitable pharmaceutical agents that can be used in conjunction with compounds of the present invention include the HMG-CoA reductase inhibitors. The HMG-CoA reductase inhibitors are agents also referred to as Statin compounds that belong to a class of drugs that lower blood cholesterol levels by inhibiting hydroxymethylglutalyl CoA (HMG-CoA) reductase. HMG-CoA reductase is the rate-limiting enzyme in cholesterol biosynthesis. The statins lower serum LDL concentrations by upregulating the activity of LDL receptors and are responsible for clearing LDL from the blood. Some representative examples the statin compounds include rosuvastatin, pravastatin and its sodium salt, simvastatin, lovastatin, atorvastatin, fluvastatin, cerivastatin, rosuvastatin, pitavastatin, BMS's "superstatin", and HMG-CoA reductase inhibitors known in the art.

Squalene synthesis inhibitors belong to a class of drugs that lower blood cholesterol levels by inhibiting synthesis of squalene. Examples of the squalene synthesis inhibitors include (S)-α-[Bis[2,2-dimethyl-1-oxopropoxy)methoxy] phosphinyl]-3-phenoxybenzenebutanesulfonic acid, mono potassium salt (BMS-188494) and squalene synthesis inhibitors known in the art.

Suitable pharmaceutical agents that can be used in conjunction with compounds of the present invention include Fibrates. Fibrate compounds belong to a class of drugs that lower blood cholesterol levels by inhibiting synthesis and secretion of triglycerides in the liver and activating a lipoprotein lipase. Fibrates have been known to activate peroxisome proliferators-activated receptors and induce lipoprotein lipase expression. Examples of fibrate compounds include bezafibrate, beclobrate, binifibrate, ciplofibrate, clinofibrate, clofibrate, clofibric acid, etofibrate, fenofibrate, gemfibrozil, nicofibrate, pirifibrate, ronifibrate, simfibrate, theofibrate, and fibrates known in the art.

LDL (low-density lipoprotein) catabolism enhancers belong to a class of drugs that lower blood cholesterol levels by increasing the number of LDL (low-density lipoprotein) receptors, examples include LDL catabolism enhancers known in the art.

Suitable pharmaceutical agents that can be used in conjunction with compounds of the present invention include the angiotensin converting enzyme (ACE) inhibitors. The angiotensin converting enzyme inhibitors belong to the class of drugs that partially lower blood glucose levels as well as lowering blood pressure by inhibiting angiotensin converting enzymes. Examples of the angiotensin converting enzyme inhibitors include captopril, enalapril, alacepril, delapril; ramipril, lisinopril, imidapril, benazepril, ceronapril, cilazapril, enalaprilat, fosinopril, moveltopril, perindopril, quinapril, spirapril, temocapril, trandolapril, and angiotensin converting enzyme inhibitors known in the art.

Suitable pharmaceutical agents that can be used in conjunction with compounds of the present invention include the insulin secretion enhancers belong to the class of drugs having the property to promote secretion of insulin from pancreatic β cells. Examples of the insulin secretion enhancers include sulfonylureas (SU). The sulfonylureas (SU) are drugs which promote secretion of insulin from pancreatic β cells by transmitting signals of insulin secretion via SU receptors in the cell membranes. Examples of the sulfonylureas include tolbutamide; chlorpropamide; tolazamide; acetohexamide; 4-chloro-N-[(1-pyrolidinylamino) carbonyl]-benzenesulfonamide (generic name: glycopyramide) or its ammonium salt; glibenclamide (glyburide); gliclazide; 1-butyl-3-metanilylurea; carbutamide; glibonuride; glipizide; gliquidone; glisoxepid; glybuthiazole; glibuzole; glyhexamide; glymidine; glypinamide; phenbutamide; tolcyclamide, glimepiride, and other insulin secretion enhancers known in the art. Other insulin secretion enhancers include N-[[4-(1-methylethyl)cyclohexyl)carbonyl]-D-phenylalanine (Nateglinide); calcium (2S)-2-benzyl-3-(cis-hexahydro-2-isoindolinylcarbonyl)propionate dihydrate (Mitiglinide, KAD-1229); and other insulin secretion enhancers known in the art.

Suitable pharmaceutical agents that can be used in conjunction with compounds of the present invention include the peroxisome proliferators-activated receptor-γ (i.e., PPAR-γ) agonists. The peroxisome proliferators-activated receptor-γ agonists represent a class of compounds that activates the nuclear receptor PPAR-γ and therefore regulate the transcription of insulin-responsive genes involved in the control of glucose production, transport and utilization. Agents in the class also facilitate the regulation of fatty acid metabolism. Examples of PPAR-γ agonists include rosiglitazone, pioglitazone, tesaglitazar, netoglitazone, GW-409544, GW-501516 and PPAR-γ agonists known in the art.

Suitable pharmaceutical agents that can be used in conjunction with compounds of the present invention include the meglitinides. The meglitinides are benzoic acid derivatives and represent a novel class of insulin secretagogues. These agents target postprandial hyperglycemia and show comparable efficacy to sulfonylureas in reducing HbAlc. Examples of meglitinides include repaglinide, nateglinide and other meglitinides known in the art.

Suitable pharmaceutical agents that can be used in conjunction with compounds of the present invention include the angiotensin II receptor antagonists. Angiotensin II receptor antagonists target the angiotensin II receptor subtype 1 (i.e., AT1) and demonstrate a beneficial effect on hypertension. Examples of angiotensin II receptor antagonists include losartan (and the potassium salt form), and angiotensin II receptor antagonists known in the art.

Other treatments for one or more of the diseases cited herein include the use of pharmaceutical agents known in the art belonging to the classes of drugs referred to, but not limited to, the following: amylin agonists (for example, pramlintide), insulin secretagogues (for example, GLP-1 agonists; exendin-4; insulinotropin (NN2211); dipeptyl peptidase inhibitors (for example, NVP-DPP-728), acyl CoA cholesterol acetyltransferase inhibitors (for example, Ezetimibe, eflucimibe, and like compounds), cholesterol absorption inhibitors (for example, ezetimibe, pamaqueside and like compounds), cholesterol ester transfer protein inhibitors (for example, CP-529414, JTT-705, CETi-1, and like compounds), microsomal triglyceride transfer protein inhibitors (for example, implitapide, and like compounds), cholesterol modulators (for example, NO-1886, and like compounds), bile acid modulators (for example, GT103-279 and like compounds) and squalene synthase inhibitors.

In some embodiments the pharmaceutical agent is selected from the group consisting of: apolipoprotein-B secretion/microsomal triglyceride transfer protein (apo-B/MTP) inhibitors, MCR-4 agonists, cholescystokinin-A (CCK-A) agonists, serotonin and norepinephrine reuptake inhibitors (for example, sibutramine), sympathomimetic agensts, β₃ adrenergic receptor agonists, dopamine agonists (for example, bromocriptine), melanocyte-stimulating hormone receptor analogs, cannabinoid 1 receptor antagonists [for example, SR141716: *N*-(piperidin-1-yl)-5-(4-chlorophenyl)-1-(2,4-dichlorophenyl)-4-methyl-1*H*-pyrazole-3-carboxamide], melanin concentrating hormone antagonists, leptons (the OB protein), leptin analogues, leptin receptor agonists, galanin antagonists, lipase inhibitors (such as tetrahydrolipstatin, i.e., Orlistat), anorectic agents (such as a bombesin agonist), Neuropeptide-Y antagonists, thyromimetic agents, dehydroepiandrosterone or an analogue thereof, glucocorticoid receptor agonists or antagonists, orexin receptor antagonists, urocortin binding protein antagonists, glucagon-like peptide-1 receptor agonists, ciliary neutrotrophic factors (such as Axokine^{™}), human agouti-related proteins (AGRP), ghrelin receptor antagonists, histamine 3 receptor antagonists or reverse agonists, neuromedin U receptor agonists, noradrenergic anorectic agents (for example, phentermine, mazindol and the like), appetite suppressants (for example, bupropion) and the like. In further embodiments, the pharmaceutical agent is selected from the group consisting of orlistat, sibutramine, bromocriptine, ephedrine, leptin, and pseudoephedrine.

In some embodiments the pharmaceutical agent is selected from the group consisting of: sulfonylureas, meglitinides, biguanides, α-glucosidase inhibitors, peroxisome proliferators-activated receptor-γ (i.e., PPAR-γ) agonists, insulin, insulin analogues, HMG-CoA reductase inhibitors, cholesterol-lowering drugs (for example, fibrates that include: fenofibrate, bezafibrate, gemfibrozil, clofibrate and the like; bile acid sequestrants which include: cholestyramine, colestipol and the like; and niacin), antiplatelet agents (for example, aspirin and adenosine diphosphate receptor antagonists that include: clopidogrel, ticlopidine and the like), angiotensin-converting enzyme inhibitors, angiotensin II receptor antagonists and adiponectin.

In accordance with the present invention, the combination can be used by mixing the respective active components either all together or independently with a physiologically acceptable carrier, excipicient, binder, diluent, etc., as described herein above, and administering the mixture or mixtures either orally or non-orally as a pharmaceutical composition. When a compound of the present invention is administered as a combination therapy with another active compound the therapeutic agents can be formulated as a separate pharmaceutical compositions given at the same time or at different times, or the therapeutic agents can be given as a single composition.

### OTHER UTILITIES

Another object of the present invention relates to radio-labeled compounds of Formula (I) that would be useful not only in radio-imaging but also in assays, both in vitro and in vivo, for localizing and quantitating the **RUP25** receptor in tissue samples, including human, and for identifying **RUP25** receptor ligands by inhibition binding of a radio-labeled compound. It is a further object of this invention to develop novel **RUP25** receptor assays of which comprise such radio-labelad compounds.

Described herein are isotopically-labeled compounds of Formula (Ia). "Isotopically" or "radio-labeled" compounds are those which are identical to compounds disclosed herein, but for the fact that one or more atoms are replaced or substituted by an atom having an atomic mass or mass number different from the atomic mass or mass number typically found in nature (i.e., naturally occurring). Suitable radionuclides that may be incorporated in compounds of the present invention include but are not limited to ²H (also written as D for deuterium), ³H (also written as T for tritium), ¹¹C, ¹³C, ¹⁴C, ¹³N, ¹⁵N, ¹³O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I. The radionuclide that is incorporated in the instant radio-labeled compounds will depend on the specific application of that radio-labeled compound. For example, for in vitro RUP25 receptor labeling and competition assays, compounds that incorporate ³H, ¹⁴C, ⁸²Br, ¹²⁵I, ¹³¹I, ³⁵S or will generally be most useful. For radio-imaging applications ¹¹C, ¹⁸F, ¹²⁵I, ¹²³I,¹²⁴I, ¹³¹I, ⁷⁵Br, ⁷⁶Br or ⁷⁷Br will generally be most useful.

It is understood that a "radio-labeled" or "labeled compound" is a compound of Formula (Ia) that has incorporated at least one radionuclide; in some embodiments the radionuclide is selected from the group consisting of ³H, ¹⁴C, ¹²⁵I, ³⁵S and ⁸²Br. Certain isotopically-labeled compounds of the present invention are useful in compound and/or substrate tissue distribution assays. In some embodiments the radionuclide ³H and/or ¹⁴C isotopes are useful in these studies. Further, substitution with heavier isotopes such as deuterium (i.e., ²H) may afford certain therapeutic advantages resulting from greater metabolic stability (e.g., increased in vivo half-life or reduced dosage requirements) and hence may be preferred in some circumstances. Isotopically labeled compounds of the present invention can generally be prepared by following procedures analogous to those disclosed in the Schemes supra and Examples infra, by substituting an isotopically labeled reagent for a non-isotopically labeled reagent. Other synthetic methods that are useful are discussed *infra*. Moreover, it should be understood that all of the atoms represented in the compounds of the invention can be either the most commonly occurring isotope of such atoms or the more scarce radio-isotope or nonradioactive isotope.

Synthetic methods for incorporating radio-isotopes into organic compounds are applicable to compounds of the invention and are well known in the art. These synthetic methods, for example, incorporating activity levels of tritium into target molecules, are as follows:
A. Catalytic Reduction with Tritium Gas - This procedure normally yields high specific activity products and requires halogenated or unsaturated precursors.
B. Reduction with Sodium Borohydride [³H] - This procedure is rather inexpensive and requires precursors containing reducible functional groups such as aldehydes, ketones, lactones, esters, and the like.
C. Reduction with Lithium Aluminum Hydride [³H] - This procedure offers products at almost theoretical specific activities. It also requires precursors containing reducible functional groups such as aldehydes, ketones, lactones, esters, and the like.
D. Tritium Gas Exposure Labeling - This procedure involves exposing precursors containing exchangeable protons to tritium gas in the presence of a suitable catalyst.
E. N-Methylation using Methyl Iodide [³H] - This procedure is usually employed to prepare O-methyl or N-methyl (³H) products by treating appropriate precursors with high specific activity methyl iodide (³H). This method in general allows for higher specific activity, such as for example, about 70-90 Ci/mmol.

Synthetic methods for incorporating activity levels of ¹²⁵I into target molecules include:
A. Sandmeyer and like reactions -This procedure transforms an aryl or heteroaryl amine into a diazonium salt, such as a tetrafluoroborate salt, and subsequently to ¹²⁵I labeled compound using Na¹²⁵I. A represented procedure was reported by Zhu, D.-G. and co-workers in J. Org. Chem. 2002, 67, 943-948.
B. Ortho ¹²⁵Iodination of phenols -This procedure allows for the incorporation of ¹²⁵I at the ortho position of a phenol as reported by Collier, T. L. and co-workers in J. Labeled Compd Radiopharm. 1999, 42, S264-S266.
C. Aryl and heteroaryl bromide exchange with ¹²⁵I- This method is generally a two step process. The first step is the conversion of the aryl or heteroaryl bromide to the corresponding tri-alkyltin intermediate using for example, a Pd catalyzed reaction [i.e. Pd(Ph₃P)4] or through an aryl or heteroaryl lithium, in the presence of a tri-alkyltinhalide or hexaalkylditin [e.g., (CH₃)₃SnSn(CH₃)₃]. A representative procedure was reported by Bas, M.-D. and co-workers in J. Labeled Compd Radiopharm. 2001, 44, S280-S282.
   A radio-labeled **RUP25** receptor compound of Formula (Ia) can be used in a screening assay to identify/evaluate compounds. In general terms, a newly synthesized or identified compound (i.e., test compound) can be evaluated for its ability to reduce binding of the "radio-labeled compound of Formula (Ia)" to the **RUP25** receptor. Accordingly, the ability of a test compound to compete with the "radio-labeled compound of Formula (Ia)" for the binding to the **R**UP25 receptor directly correlates to its binding affinity.

The labeled compounds of the present invention bind to the RUP25 receptor. In one embodiment the labeled compounds has an IC₅₀ less than about 500 µM, in another embodiment the labeled compound has an IC₅₀ less than about 100 µM, in yet another embodiment the labeled compound has an IC₅₀ less than about 10 µM, in yet another embodiment the labeled compound has an IC₅₀ less than about 1 µM, and in still yet another embodiment the labeled inhibitor has an IC₅₀ less than about 0.1 µM.

Other uses of the disclosed receptors and methods will become apparent to those in the art based upon, inter alia, a review of this disclosure.

As will be recognized, the steps of the methods of the present invention need not be performed any particular number of times or in any particular sequence. Additional objects, advantages, and novel features of this invention will become apparent to those skilled in the art upon examination of the following examples thereof, which are intended to be illustrative and not intended to be limiting.

### EXAMPLES

The following Examples are provided for illustrative purposes and not as a means of limitation. One of ordinary skill in the art would be able to design equivalent assays and methods based on the disclosure herein.

### Exampl 1

### RODENT DIABETES MODELS

Rodent models of type 2 diabetes associated with obesity and insulin resistance have been developed. Genetic models such as db/db and ob/ob [*see* Diabetes (1982) 31:1-6] in mice and fa/fa in Zucker rats have been developed for understanding the pathophysiology of disease and for testing candidate therapeutic compounds [Diabetes (1983) 32:830-838; Annu Rep Sankyo Res Lab (1994) 46:1-57]. The homozygous animals, C57 BL/KsJ-db/db mice developed by Jackson Laboratory are obese, hyperglycemie, hyperinsulinemie and insulin resistant [J Clin Invest (1990) 85:962-967], whereas heterozygotes are lean and normoglycemie. In the db/db model, mice progressively develop insulinopenia with age, a feature commonly observed in late stages of human type 2 diabetes when sugar levels are insufficiently controlled. Since this model resembles that of human type 2 diabetes, the compounds of the present invention are tested for activities including, but not limited to, lowering of plasma glucose and triglycerides. Zucker (fa/fa) rats are severely obese, hyperinsulinemie, and insulin resistant {Coleman, Diabetes (1982) 31:1; E Shafrir in Diabetes Mellitus, H Rifkin and D Porte, Jr, Eds [Elsevier Science Publishing Co, New York, ed. 4, (1990), pp. 299-340]}, and the fa/fa mutation may be the rat equivalent of the murine db mutation [Friedman et al, Cell (1992) 69:217-220; Truett et al, Proc Nad Acad Sci USA (1991) 88:7806]. Tubby (tub/tub) mice are characterized by obesity, moderate insulin resistance and hyperinsulinemia without significant hyperglycemia [Coleman et al. Heredity (1990) 81:424].

Described herein is use of compounds of the invention for reducing the insulin resistance and hyperglycemia in any or all of the above rodent diabetes models, in humans with type 2 diabetes or other preferred metabolic-related disorders or disorders of lipid metabolism described previously, or in models based on other mammals. Plasma glucose and insulin levels will be tested, as well as other factors including, but not limited to, plasma free fatty acids and triglycerides.

### In Vivo Assay for Anti-Hyperglycemic Activity of Compounds of the Invention

Genetically altered obese diabetic mice (db/db) (male, 7-9 weeks old) are housed (7-9 mice/cage) under standard laboratory conditions at 22°C and 50% relative humidity, and maintained on a diet of Purina rodent chow and water *ad libitum.* Prior to treatment, blood is collected from the tail vein of each animal and blood glucose concentrations are determined using One Touch Basic Glucose Monitor System (Lifescan). Mice that have plasma glucose levels between 250 to 500 mg/dL are used. Each treatment group consists of seven mice that are distributed so that the mean glucose levels are equivalent in each group at the start of the study. db/db mice are dosed by micro-osmotic pumps, inserted using isoflurane anesthesia, to provide compounds of the invention, saline, or an irrelevant compound to the mice subcutaneously (s.c.). Blood is sampled from the tail vein at intervals thereafter and analyzed for blood glucose concentrations. Significant differences between groups (comparing compounds of the invention to saline-treated) are evaluated using Student t-test.

### Example 2

### MOUSE A THEROSCLEROSIS MODEL

Adiponcctin-deficient mice generated through knocking out the adiponectin gene have been shown to be predisposed to atherosclerosis and to be insulin resistant. The mice are also a suitable model for ischemic heart disease [Matsuda, M et al. J Biol Chem (2002) July, and references cited therein].

Adiponectin knockout mice are housed (7-9 mice/cage) under standard laboratory conditions at 22°C and 50% relative humidity. The mice are dosed by micro-osmotic pumps, inserted using isoflurane anesthesia, to provide compounds of the invention, saline, or an irrelevant compound to the mice subcutaneously (s.c.). Neointimal thickening and ischemic heart disease are determined for different groups of mice sacrificed at different time intervals. Significant differences between groups (comparing compounds of the invention to saline-treated) are evaluated using Student t-test

### Example 3

### In Vitro Biological Activity

A modified Flash Plate^{™} Adenylyl Cyclase kit (New England Nuclear; Cat. No. SMP004A) was utilized for direct identification of candidate compounds as agonists to h**RUP25** (Seq. Id. Nos. 1 & 2) in accordance with the following protocol:

CHO cells stably transfected with an expression vector encoding hRUP25 and cultured under condition permissive for cell surface expression of the encoded hRUP25 receptor were harvested from flasks *via* non-enzymatic means. The cells were washed in PBS and resuspended in the manufacturer's Assay Buffer. Live cells were counted using a hemacytometer and Trypan blue exclusion, and the cell concentration was adjusted to 2x10⁶ cells/mL. cAMP standards and Detection Buffer (comprising 2 µCi of tracer [¹²⁵I]-cAMP (100 µL) to 11 mL Detection Buffer) were prepared and maintained in accordance with the manufacturer's instructions. Candidate compounds identified as per above (if frozen, thawed at room temperature) were added to their respective wells (preferably wells of a 96-well plate) at increasing concentrations (3µL/well; 12µM final assay concentration). To these wells, 100,000.cells in 50µL of Assay Buffer were added and the mixture was then incubated for 30 minutes at room temperature, with gentle shaking. Following the incubation, 100µL of Detection Buffer was added to each well, followed by incubation for 2-24 hours. Plates were counted in a Wallae MicroBeta^{™} plate reader using "Prot. #31" (as per manufacture instructions).

### Example 4: Representative Biological Activity.

The biological *in vitro* activity was determined using the cAMP Whole Cell method, one representative example is shown in the table below:

| Compound No. | hRUP25 (IC₅₀) cAMPWhole Cell (µM) |
|---|---|
| 8 | 0.90* |

| | |
|---|---|
| *Value is an average of two (2) trials. | |

### Example 5: Syntheses Of Compounds Of The Invention.

¹H and ¹³C NMR were referenced to residual solvent peaks. ¹⁹F NMR was referenced to 1,2,3,4.5,6,-hexafluorobenzene, at -164.0 ppm.

### Reference Example 5.1: Preparation of 4-Fluoro-5-methyl-1H-pyrazole-3-carboxylic add ethyl ester (Compound 1).

Ethyl-5-methylpyrazole-3-carboxylate (1.025g, 6.65mmol) was taken up in acetonitrile (200cm³) and selectfluor_{™} (2.40g, 6.77mmol) added. The resulting solution was heated to 65°C for 18 hours in a sealed polypropylene flask, solvent was removed under reduced pressure, and the resulting solid taken up in dichloromethane (150cm³) and washed with 3M aqueous hydrochloric acid (100cm³). Solvent was removed under reduced pressure and the residual solid purified by column chromatography (dichloromethane, to 15% EtOAc/dichloromethane, silica) to give ethyl-4-fluoro-5-methylpyrazole-3-carboxylate as an off white solid (0.327g, 1.9mmol, 29%).
m/z (ES⁻): 171.0[M-H]⁻. ¹H NMA (CDCl₃):4.41 (q,2H.J=7.1, OCH₂CH₃), 2.31(s, 3H, CH₃), 1.38 (t, 3H, J=7.1, OCH₂CH₃). ¹³C NMR (CDCl₃): 159.8 (CO₂Et), 148.8, 1462, 128 (br d, J=765, C-F), 61.4, 14.1, 8.8.

### Example 5.2: Preparation of 4-Fluoro-5-methyl-2H-pyrazole-3-carboxylic acid (Compound 2).

Ethyl-4-fluoro-5-methylpyrazole-3-carboxylate (0.44g, 2.56mmol) was taken up in a solution of 1:5:1 MeOH:THF:1M aq LiOH (70cm³) and heated to 80°C for 3 hours. Solvent was removed under reduced pressure, and the resulting solid taken up in water (50cm³), acidified to pH 1 by the addition of 1M aqueous hydrochloric acid and extracted into ethyl acetate (100cm³). Solvent was removed under reduced pressure to give 4-fluoro-5-methylpyrazole-3-carboxylate as an off white solid (0.354g, 2.45mmol, 96%). m/z (ES⁻): 142.9 [M-H]⁻. ¹H NMR (CD₃OD): 2.06 (s, 3H, CH₃). ¹³C NMR (CD₃OD): 163.4(CO₂H)), 149.9(d, J=315, C-F), 133 (br), 128(br), 9.1. ¹⁹F NMR (CD₃OD): -174.

### Reference Example 53: 5-Cyclopropyl-4-fluoro-1H-pyrazole-3-carboxylic acid ethyl ester (Compound 3).

¹H NMR (CDCl₃): 4.40 (q, 2H, J=7.1, OCH₂CH₃),2.0-1.8 (m,1H, CH-[CH₂CH₂]-), 1.39 (t, 3H, J=7.1, OCH₂CH₃), 1.0-0.8 (m, 4H, CH-[CH₂CH₂]-).

### Example 5.4: 5-Cyclopropyl-4-fluoro-1H-pyrazole-3-carboxylic add (Compound 4).

¹H NMR (CD₃OD): 1.95-1.85(m,1H,CH-[CH₂CH₂]-),1.05-0.95 (m, 2H), 0.90-0.85 (m, 2H). ¹³C NMR (CD₃OD): 162.5 (C₌O), 148.7 (d, J=254, C-F),137.4 (d, J=15), 126.9 (d, J=13), 6.6 (CH₂),5.7 (CH). ¹⁹F NMR (CD₃OD): -173.6.

### Reference Example 5.5: 5-Ethyl-4-fluoro-1H-pyrazolo-3-carboxylic acid ethyl ester (Compound 5).

¹H NMR (CDCl₃); 4.41 (q, 2H, J=7.1, OCH₂CH₃). 2.71 (q, 2H, J=7.6, CH₂CH₃),1.40 (t, 3H, J=7.1,OCH₂CH₃), 1.27 (t, 3H, J=7.6, CH₂CH₃).

### Example 5.6: 5-Ethyl-4-fluoro-1H-pyrazole-3-carboxylic add (Compound 6).

¹H NMR (CD₃OD): 2.47 (q, 2H, J=7.6, CH₂CH₃), 1.06 (t, 2H, J=7.6, CH₂CH₃). ¹³C NMR (CD₃OD): 162.7 (C=O), 148.3 (d, J=252, C-F), 137.1 (d, J=18),127.2 (d, J=11), 18.0 (CH₂),132 (CH₃). ¹⁹F NMR (CD₃OD): -172.5.

### Reference Example 5.7: 5-Butyl-4-fluoro-1H-pyrazole-3-carboxylic acid ethyl ester (Compound 7).

¹H NMR (CDCl₃): 4.41 (q, 2H, J=7.1. OCH₂CH₃), 2.67 (t, 2H, J=7.6, CH₂CH₃),1.65 (quintet like, 2H, J=7.6, (CH₂C₂H₅), 1.42-1.35 (m, 5H, OCH₂CH₃ & CH₂CH₃), 0.94 (t, 3H, J=7.4, CH₃).

### Example 5.8: 5-Butyl-4-fluoro-1H-pyrazole-3-carboxylic acid (Compound 8).

¹H NMR (CD₃OD): 2.44 (t, 2H, J=7.6, CH₂CH₇), 1.43 (quintet like, 2H, J=7.6, (CH₂C₂H5), 1.16 (sextet like, 2H, J=7.5, CH₂CH₃), 0.74 (t, 3H, J=7.4, CH₃). ¹³C NMR (CD₃OD): 162.7 (C=O), 148.6 (d, J=252, C-F), 135.7 (d, J=19),127.3 (d, J=10). 31.6 (CH₂C₂H₅), 24.1 (CH₂C₃H₇), 23.2 (CH₂CH₃), 14.0 (CH₃). ¹⁹F NMR (CD₃OD): -173.5.

### Reference Example 5.9: 5-(1,1-Difluoro-ethyl)-1H-pyrazole-3-carboxylic acid ethyl ester (Compound 9).

5-Acetyl-1H-pyrazole-3-carboxylic acid ethyl ester (1.04g, 5.71 mmol) was taken up in dichloromethane (50 cm³) and chilled (0°C). DAST (3.22g, 20.0 mmol) was added and the solution stirred under argon at 0°C for a further 10 minutes and at 35°C for 18 hours. Further DAST (0.93, 5.71mmol) was added and the solution stirred for a further 30 minutes at 40°C. The resulting solution was washed with water (40 cm³), solvent removed under reduced pressure and the residual solid purified by column chromatography (5-25% EtOAc/n-hexane, silica) to give 5-(1',1'-Difluoro-ethyl)-1H-pyrazole-3-carboxylic acid ethyl ester as a yellow solid (0.94g, 81%). ¹HNMR(CD₃OD): 6.96 (s, 1H,pyr-H),4.38(q,2H, J=7.1, OCH₂CH₃), 1.99(t,3H, J=18.4, CF₂CH₃), 1.38 (t, 3H, J=7.1, OCH₂CH₃).

The intermediate 5-acetyl-1H-pyrazole-3-carboxylic acid ethyl ester was prepared in the following manner:
3,3-Dimethoxy-2-butanone (2.64g, 20.0mmol) and diethyl oxalate (2.92g, 20.0mmol) were taken up in ethanol (50 cm³) and sodium ethoxide (21% in EtOH, 9.0cm³, 25.6mmol) added. The resulting solution was heated under argon at 75°C for 2 hours then allowed to cool to room temperature. Hydrazine monohydrochloride (1.50g, 22.0mmol) and water (5 cm³) were added and the solution for a further 2 hours under argon at 75°C for 2 hours. Solvent was removed under reduced pressure and the residual oil purified by preparative HPLC to give 5-acetyl-1H-pyrazole-3-carboxylic acid ethyl ester (0.95g, 26%). m/z(ES⁺):183.0[M+H]^{+ 1}H NMR (CDCl₃): 7.32(s, 1H, pyrazole-H), 4.43 (q, 2H, J=7.1, OCH₂CH₃), 2.61 (s, 3H, COCH₃), 1.41 (t, 3H,J=7.1, OCH₂CH₃).

### Example 5.10: General procedure for the preparation of pyrazoles of the invention:

To a corresponding ketone dissolved in ethanol (5mL/mmol), is added diethyl oxalate (1.2eq.) and 1M solution *t*-BuOK inTHF(1.1eq.). The mixture was heated at 75°C for 30 minutes, then cooled to 4°C in a ice bath. An aqueous solution of Hydrazine (2eq., 2mL/mmoL) was added and the resulting mixture was heated at 75°C for 1 hour. Ethanol was removed under reduced pressure and the crude was diluted with a saturated aqueous solution of NaHCO₃ and extracted with EtOAc. The organic layer was dried over Na₂SO₄ and concentrated to give the pirazole ester derivative. The hydrolysis of the ester function was then performed under basic condition using 5N aqueous solution NaOH at 95°C over a period of 2h. The pH of the solution was adjusted to 1 using a 12N HCl and mixture was extracted with AcOEt or Acetonitrile, the organic layer was dried over Na₂SO₄ and concentrated. The crude material was purified by HPLC to afford the acid derivative.

### Reference Example 5.11:5-(3-Fluoro-benzyl)-2H-pyrazole-3-carboxylic acid (Compound 10).

5-(3-Fluoro-benzyl)-2H-pyrazole-3-carboxylic acid was prepared using the general procedure as described in Example 5.10. ¹HNMR (DMSO,400MHz) δ (ppm): 7.34 (1H, m), 7.07 (2H, m), 6.50 (1H,s), 3.98 (2H, s). Mass Spectrum: m/z: 221 (M+1)⁺.

### Reference Example 5.12: 5-(3-Bromo-benzyl)-2H-pyrazole-3-carboxylic acid (Compound 11).

5-(3-Bromo-benzyl)-2H-pyrazole-3-carboxylic acid was prepared using the general procedure as described in Example 5.10. ¹HNMR(DMSO,400MHz)δ(ppm):7.46(1H,s). 7.42 (1H, m), 727 (2H, m), 6.51 (1H,s), 3.97 (2H, s). Mass Spectrum: m/z: 281 (M+1)+, 283 (M+1)⁺.

### Reference Example 5.13: 5-(4-Bromo-benzyl)-2H-pyrazole-3-carboxylic acid (Compound 12).

5-(4-Bromo-benzyl)-2H-pyrazole-3-carboxylic acid was prepared using the general procedure as described in Example 5.10.; ¹H NMR (DMSO, 400MHz) δ (ppm): 7.48 (2H, d, J = 8.3Hz), 7.2 (2H, d, J = 8.3Hz), 6.47 (1H,s), 3.94 (2H, s). Mass Spectrum: m/z: 281 (M+1)⁺, 283 (M+1)⁺.

### Reference Example 5.14: 5-[2-(4-Methoxy-phenyl)-ethyl]-2H-pyrazole-3-carboxylic add (Compound 13).

5-[2-(4-Methoxy-phenyl)-ethyl]-2H-pyazole-3-carboxylic acid was prepared using the general procedure as described in Example 5.10. ¹H NMR (DMSO, 400MHz) 8 (ppm): 12.9 (1H, broad s), 7.12 (2H, d, J= 8.5Hz), 6.83 (2H, d, J= 8.5Hz), 6.47 (1H, s),3.71 (2H, s), 2.86 (2H, s). Mass Spectrum: m/z: 247 (M+1)⁺.

Although a variety of expression vectors are available to those in the art, for purposes of utilization for both the endogenous and non-endogenous human GPCRs, it is most preferred that the vector utilized be pCMV. This vector was deposited with the American Type Culture Collection (ATCC) on October 13, 1998 (10801 University Blvd., Manassas, VA 20110-2209 USA) under the provisions of the Budapest Treaty for the International Recognition of the Deposit of Microorganisms for the Purpose of Patent Procedure. The DNA was tested by the ATCC and determined to be viable. The ATCC has assigned the following deposit number to pCMV: ATCC#203351.

## Claims

1. A compound selected from compounds of Formula (**Ia**) and pharmaceutically acceptable salts, solvates, and hydrates thereof: 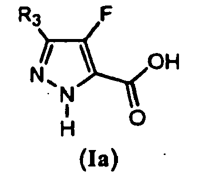 wherein:
R₃ is C₃₋₆ cycloalkyl, C₁₋₁₂ alkyl, C₁₋₁₂ haloalkyl, C₃₋₆ cycloalkyl-C₁₋₄-alkylene, aryl-C₁₋₄-alkylene or heteroaryl-C₁₋₄-alkylene, wherein said aryl-C₁₋₄-alkylene and heteroaryl-C₁₋₄-alkylene can be optionally substituted with 1 to 5 substituents selected from C₁₋₄ acyl, C₁₋₄ acyloxy, C₂₋₄-alkenyl, C₁₋₄ alkoxy, C₁₋₄ alkyl, C₁₋₄ alkylcarboxamide, C₂₋₄ alkynyl, C₁₋₄ alkylsulfonamide, C₁₋₄ alkylsulfinyl, C₁₋₄ alkylsulfonyl, C₁₋₄ alkylthio, C₁₋₄ alkylureyl, amino, C₁₋₄ alkylamino, C₁₋₄ dialkylamino, arylsulfonyl, carbo-C₁₋₄-alkoxy, carboxamide, carboxy, cyano, C₃₋₆ cycloalkyl, C₁₋₄ dialkylcarboxamide, C₁₋₄ dialkylsulfonamide, halogen, C₁₋₄ haloalkoxy, C₁₋₄ haloalkyl, C₁₋₄ haloalkylsulfinyl, C₁₋₄ haloalkylsulfonyl, C₁₋₄ haloalkylthio, heterocyclyl, hydroxyl, thio, nitro, C₄₋₆oxo-cycloalkyl, sulfonamide, and sulfonic acid;
wherein:
"aryl" denotes an aromatic ring radical containing 6 to 10 ring carbons;
"heteroaryl" denotes an aromatic ring system that may be a single ring, two fused rings or three fused rings wherein at least one ring carbon is replaced with a heteroatom selected from O, S and N, wherein the N is optionally substituted with H, C₁₋₄ acyl, or C₁₋₄ alkyl;
"heterocyclyl" denotes a 3-, 4-, 5-, 6- or 7-member non-aromatic carbon ring wherein one, two or three ring carbons are replaced by a heteroatom selected from O, S, N, wherein the N is optionally substituted with H, C₁₋₄ acyl or C₁₋₄ alkyl, and ring carbon atoms are optionally substituted with oxo or thiooxo; and
"carbo-C₁₋₄-alkoxy" denotes a C₁₋₄ alkyl ester of a carboxylic acid.

2. A compound according to claim 1, wherein R₃ is C₁₋₁₂ alkyl.

3. A compound according to claim 1, wherein R₃ is -CH₃, -CH₂CH₃, -(CH₂)₂CH₃, -(CH₂)₃CH₃, -(CH₂)₄CH₃, -(CH₂)₅CH₃, -(CH₂)₆CH₃, -(CH₂)₇CH_{3,} -(CH₂)₈CH₃, -(CH₂)₉CH₃, -(CH₂)₁₀CH₃, or -(CH₂)₁₁CH₃.

4. A compound according to claim 1, wherein R₃ is -CH₃, -CH₂CH₃, -(CH₂)₂CH₃, or -(CH₂)₃CH₃.

5. A compound according to claim 1, wherein R₃ is C₁₋₁₂ haloalkyl.

6. A compound according to claim 1, wherein R₃ is -CHF₂, -CH₂F, -CH₂CF₃, -CH₂CHF₂, -CF₂CH₃; -CH₂CH₂CF₃, -CH₂CF₂CH₃, -CH₂CF₂CF₃. -CH₂CH₂CH₂CHF₂, or -CH₂CH₂CF₂CH₃.

7. A compound according to claim 1, wherein R₃ is -CF₃, -CF₂CF₃, or -(CF₂)₂CF₃.

8. A compound according to claim 1, wherein R₃ is C₃₋₆ cycloalkyl.

9. A compound according to claim 1, wherein R₃ is cyclopropyl.

10. A compound according to claim 1, wherein R₃ is C₃₋₆ cycloalkyl-C₁₋₄-alkylene optionally substituted with 1 to 5 substituents selected from C₁₋₄ alkoxy, C₁₋₄ alkyl, C₁₋₄ alkylsulfonyl, C₁₋₄ alkylthio, carboxamide, carboxy, cyano, halogen, C₁₋₄ haloalkoxy, C₁₋₄ haloalkyl, C₁₋₄ haloalkylsulfonyl, C₁₋₄ haloalkylthio, hydroxyl, thio, nitro, and sulfonamide.

11. A compound according to claim 10, wherein said C₃₋₆ cycloalkyl-C₁₋₄-alkylene is selected from cyclopropylmethyl, 2-cyclopropyl-ethyl, 1-cyclopropyl-ethyl, 3-cyclopropyl-propyl, 2-cyclopropyl-propyl, 1-cyclopropyl-propyl, 1-cyclopropyl-1-methyl-ethyl, and 2-cyclopropyl-1-methyl-ethyl.

12. A compound according to claim 11, wherein said C₃₋₆ cycloalkyl-C₁₋₄-alkylene is cyclopropylmethyl.

13. A compound according to claim 1, wherein R₃ is aryl-C₁₋₄-alkylene optionally substituted with 1 to 5 substituents selected from C₁₋₄ alkoxy, C₁₋₄ alkyl, C₁₋₄ alkylsulfonyl, C₁₋₄ alkylthio, carboxamide, carboxy, cyano, halogen, C₁₋₄ haloalkoxy, C₁₋₄ haloalkyl, C₁₋₄ haloalkylsulfonyl, C₁₋₄ haloalkylthio, hydroxyl, thio, nitro, and sulfonamide.

14. A compound according to claim 13, wherein said aryl-C₁₋₄-alkyleneis selected from benzyl, phenethyl, 1-phenyl-ethyl, 3-phenyl-propyl, 2-phenyl-propyl, 1-phenyl-propyl, 1-phenyl-1-methyl-ethyl, and 2-phenyl-1-methyl-ethyl.

15. A compound according to claim 13, wherein said aryl-C₁₋₄-alkylene is benzyl.

16. A compound according to claim 1, wherein R₃ is heteroaryl-C₁₋₄-alkylene optionally substituted with 1 to 5 substituents selected from C₁₋₄ alkoxy, C₁₋₄ alkyl, C₁₋₄ alkylsulfonyl, C₁₋₄ alkylthio, carboxamide, carboxy, cyano, halogen, C₁₋₄ haloalkoxy, C₁₋₄ haloalkyl, C₁₋₄ haloalkylsulfonyl, C₁₋₄ haloalkylthio, hydroxyl, thio, nitro, and sulfonamide.

17. A compound according to claim 16, wherein said heteroaryl-C₁₋₄-alkylene is selected from thiophen-2-yl-methyl, thiophen-3-yl-methyl, pyrrol-1-yl-methyl, pyrrol-2-yl-methyl, pyrrol-3-yl-methyl, furan-2-yl-methyl, furan-3-yl-methyl, 2-thiophen-2-yl-ethyl, 2-thiophen-3-yl-ethyl, 2-furan-2-yl-ethyl, 2-furan-3-yl-ethyl, 2-pyrrol-1-yl-ethyl, 2-pyrrol-2-yl-ethyl, and 2-pyrrol-3-yl-ethyl.

18. A compound according to claim 16, wherein said heteroaryl-C₁₋₄-alkylene is selected from thiophen-2-yl-methyl, thiophen-3-yl-methyl, pyrrol-1-yl-methyl, pyrrol-2-yl-methyl, pyrrol-3-yl-methyl, furan-2-yl-methyl, and furan-3-yl-methyl.

19. A compound according to claim 1, wherein said compound is selected from the following compounds and pharmaceutically acceptable salts, solvates, and hydrates thereof:
4-Fluoro-5-methyl-2*H*-pyrazole-3-carboxylic acid;
5-Difluoromethyl-4-fluoro-2*H*-pyrazole-3-carboxylic acid;
5-(2,2-Difluoro-ethyl)-4-fluoro-2H-pyrazole-3-carboxylic acid;
5-(1,1-Difluoro-ethyl)-4-fluoro-2*H*-pyrazole-3-carboxylic acid;
5-(2,2-Difluoro-propyl)-4-fluoro-2*H*-pyrazole-3-carboxylic acid;
5-Ethyl-4-fluoro-2*H*-pyrazole-3-carboxylic acid;
4-Fluoro-5-propyl-2*H*-pyrazole-3-carboxylic acid;
5-Butyl-4-fluoro-2*H*-pyrazole-3-carboxylic acid;
4-Fluoro-5-pentyl-2*H*-pyrazole-3-carboxylic acid;
4-Fluoro-5-hexyl-2*H*-pyrazole-3-carboxylic acid;
4-Fluoro-5-heptyl-2*H*-pyrazole-3-carboxylic acid;
4-Fluoro-5-octyl-2*H*-pyrazole-3-carboxylic acid;
4-Fluoro-5-nonyl-2*H*-pyrazole-3-carboxylic acid;
5-Decyl-4-fluoro-2*H*-pyrazole-3-carboxylic acid;
4-Fluoro-5-undecyl-2*H*-pyrazole-3-carboxylic acid;
5-Dodecyl-4-fluoro-2*H*-pyrazole-3-carboxylic acid;
5-Cyclopropyl-4-fluoro-2*H*-pyrazole-3-carboxylic acid; and
5-Cyclopropylmethyl-4-fluoro-2*H*-pyrazole-3-carboxylic acid.

20. A pharmaceutical composition comprising a compound according to any one of claims 1 to 19 in combination with a pharmaceutically acceptable carrier.

21. A pharmaceutical composition according to claim 20, further comprising an agent selected from α-glucosidase inhibitor, aldose reductase inhibitor, biguanide, HMG-CoA reductase inhibitor, squalene synthesis inhibitor, fibrate, LDL catabolism enhancer, angiotensin converting enzyme inhibitor, insulin secretion enhancer, and thiazolidinedione.

22. A compound according to any one of claims 1 to 19 for use in a method of treatment of the human or animal body by therapy.

23. A compound according to any one of claims 1 to 19 for use in a method of treatment of a metabolic-related disorder.

24. Use of a compound according to any one of claims 1 to 19 in the manufacture of a medicament for use in the treatment of a metabolic-related disorder.

25. A compound according to any one of claims 1 to 19 for use in the treatment of dyslipidemia, atherosclerosis, coronary heart disease, insulin resistance, obesity, impaired glucose tolerance, atheromatous disease, hypertension, stroke, Syndrome X, heart disease, or type 2 diabetes.

26. Use of a compound according to any one of claims 1 to 19 in the manufacture of a medicament for use in the treatment of dyslipidemia, atherosclerosis, coronary heart disease, insulin resistance, obesity, impaired glucose tolerance, atheromatous disease, hypertension, stroke, Syndrome X, heart disease, or type 2 diabetes.

27. A compound according to any one of claims 1 to 19 for use in the treatment of dyslipidemia, atherosclerosis, coronary heart disease, insulin resistance, or type 2 diabetes.

28. Use of a compound according to any one of claims 1 to 19 in the manufacture of a medicament for use in the treatment of dyslipidemia, atherosclerosis, coronary heart disease, insulin resistance, or type 2 diabetes.

29. A compound according to any one of claims 1 to 19 for use in the treatment of atherosclerosis.

30. Use of a compound according to any one of claims 1 to 19 in the manufacture of a medicament for use in the treatment of atherosclerosis.

## Patentansprüche

1. Verbindung, die aus Verbindungen der Formel (Ia) und pharmazeutisch annehmbaren Salzen, Solvaten und Hydraten davon ausgewählt ist: 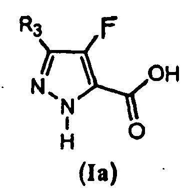 worin:
R₃ = C₃₋₆-Cycloalkyl, C₁₋₁₂-Alkyl, C₁₋₁₂-Halogenalkyl, C₃₋₆-Cycloalkyl-C₁₋₄-alkylen, Aryl-C₁₋₄-alkylen oder Heteroaryl-C₁₋₄-alkylen ist, worin das Aryl-C₁₋₄-alkylen und das Heteroaryl-C₁₋₄-alkylen gegebenenfalls mit 1 bis 5 Substituenten substituiert sind, die aus C₁₋₄-Acyl, C₁₋₄-Acyloxy, C₂₋₄-Alkenyl, C₁₋₄-Alkoxy, C₁₋₄-Alkyl, C₁₋₄-Alkylcarboxamid, C₂₋₄-Alkinyl, C₁₋₄-Alkylsulfonamid, C₁₋₄-Alkylsulfinyl, C₁₋₄-Alkylsulfonyl, C₁₋₄-Alkylthio, C₁₋₄-Alkylureyl, Amino, C₁₋₄-Alkylamino, C₁₋₄-Dialkylamino, Arylsulfonyl, Carbo-C₁₋₄-alkoxy, Carboxamid, Carboxy, Cyano, C₃₋₆-Cycloalkyl, C₁₋₄-Dialkylcarboxamid, C₁₋₄-Dialkylsulfonamid, Halogen, C₁₋₄-Halogenalkoxy, C₁₋₄-Halogenalkyl, C₁₋₄-Halogenalkylsulfinyl, C₁₋₄-Halogenalkylsulfonyl, C₁₋₄-Halogenalkylthio, Heterocyclyl, Hydroxyl, Thio, Nitro, C₄₋₆-Oxocycloalkyl, Sulfonamid und Sulfonsäure ausgewählt sind;
worin:
"Aryl" den Rest eines aromatischen Rings mit 6 bis 10 Ringkohlenstoffatomen bezeichnet;
"Heteroaryl" ein aromatisches Ringsystem bezeichnet, bei dem es sich um einen einzelnen Ring, zwei kondensierte Ringe oder drei kondensierte Ringe handeln kann, worin zumindest ein Ringkohlenstoffatom durch ein aus O, S und N ausgewähltes Heteroatom ersetzt ist, worin N gegebenenfalls mit H, C₁₋₄-Acyl oder C₁₋₄-Alkyl substituiert ist;
"Heterocyclyl" einen 3-, 4-, 5-, 6- oder 7-gliedrigen, nichtaromatischen Kohlenstoffring bezeichnet, worin ein, zwei oder drei Ringkohlenstoffatome durch ein aus O, S und N ausgewähltes Heteroatom ersetzt sind, worin N gegebenenfalls mit H, C₁₋₄-Acyl oder C₁₋₄-Alkyl substituiert ist, und die Ringkohlenstoffatome gegebenenfalls mit Oxo oder Thioxo substituiert sind, und
"Carbo-C₁₋₄-alkoxy" einen C₁₋₄-Alkylester einer Carbonsäure bezeichnet.

2. Verbindung nach Anspruch 1, worin R₃ = C₁₋₁₂-Alkyl ist.

3. Verbindung nach Anspruch 1 worin R₃ = -CH₃, -CH₂CH₃, -(CH₂)₂CH₃, -(CH₂)₃CH₃, -(CH₂)₄CH₃, -(CH₂)₅CH₃, -(CH₂)₆CH₃, -(CH₂)₇CH₃, -(CH₂)₈CH₃, -(CH₂)₉CH₃, -(CH₂)₁₀CH₃ oder -(CH₂)₁₁CH₃ ist.

4. Verbindung nach Anspruch 1, worin R₃ = -CH₃, -CH₂CH₃, -(CH₂)₂CH₃ oder -(CH₂)₃CH₃ ist.

5. Verbindung nach Anspruch 1, worin R₃ = C₁₋₁₂-Hatogenalkyl ist.

6. Verbindung nach Anspruch 1, worin R₃ = -CHF₂, -CH₂F, -CH₂CF₃, -CH₂CHF₂, -CF₂CH₃, -CH₂CH₂CF₃, -CH₂CF₂CH₃, -CH₂CF₂CF₃, -CH₂CH₂CH₂CHF₂ oder -CH₂CH₂CF₂CH₃ ist.

7. Verbindung nach Anspruch 1, worin R₃ = -CF₃, -CF₂CF₃ oder -(CF₂)₂CF₃ ist.

8. Verbindung nach Anspruch 1, worin R₃ = C₃₋₆-Cycloalkyl ist.

9. Verbindung nach Anspruch 1, worin R₃ = Cyclopropyl ist.

10. Verbindung nach Anspruch 1, worin R₃ = C₃₋₆-Cycloalkyl-C₁₋₄-alkylen ist, das gegebenenfalls mit 1 bis 5 Substituenten substituiert ist, die aus C₁₋₄-Alkoxy, C₁₋₄-Alkyl, C₁₋₄-Alkylsulfonyl, C₁₋₄-Alkylthio, Carboxamid, Carboxy, Cyano, Halogen, C₁₋₄-Halogenalkoxy, C₁₋₄-Halogenalkyl, C₁₋₄-Halogenalkylsulfonyl, C₁₋₄-Halogenalkylthio, Hydroxyl, Thio, Nitro und Sulfonamid ausgewählt sind.

11. Verbindung nach Anspruch 10, worin das C₃₋₆-Cycloalkyl-C₁₋₄-alkylen aus Cyclopropylmethyl, 2-Cyclopropylethyl, 1-Cyclopropylethyl, 3-Cyclopropylpropyl, 2-Cyclopropylpropyl, 1-Cyclopropylpropyl, 1-Cyclopropyl-1-methylethyl und 2-Cyclopropyl-1-methylethyl ausgewählt ist.

12. Verbindung nach Anspruch 11, worin das C₃₋₆-Cycloalkyl-C₁₋₄-alkylen Cyclopropylmethyl ist.

13. Verbindung nach Anspruch 1, worin R₃ = Aryl-C₁₋₄-alkylen ist, das gegebenenfalls mit 1 bis 5 Substituenten substituiert ist, die aus C₁₋₄-Alkoxy, C₁₋₄-Alkyl, C₁₋₄-Alkylsulfonyl, C₁₋₄-Alkylthio, Carboxamid, Carboxy, Cyano, Halogen, C₁₋₄-Halogenalkoxy, C₁₋₄-Halogenalkyl, C₁₋₄-Halogenalkylsulfonyl, C₁₋₄-Halogenalkylthio, Hydroxyl, Thio, Nitro und Sulfonamid ausgewählt sind.

14. Verbindung nach Anspruch 13, worin das Aryl-C₁₋₄-alkylen aus Benzyl, Phenethyl, 1-Phenylethyl, 3-Phenylpropyl, 2-Phenylpropyl, 1-Phenylpropyl, 1-Phenyl-1-methylethyl und 2-Phenyl-1-methylethyl ausgewählt ist.

15. Verbindung nach Anspruch 13, worin das Aryl-C₁₋₄-alkylen Benzyl ist.

16. Verbindung nach Anspruch 1, worin R₃ = Heteroaryl-C₁₋₄-alkylen ist, das gegebenenfalls mit 1 bis 5 Substituenten substituiert ist, die aus C₁₋₄-Alkoxy, C₁₋₄-Alkyl, C₁₋₄-Alkylsulfonyl, C₁₋₄-Alkylthio, Carboxamid, Carboxy, Cyano, Halogen, C₁₋₄-Halogenalkoxy, C₁₋₄-Halogenalkyl, C₁₋₄-Halogenalkylsulfonyl, C₁₋₄-Halogenalkylthio, Hydroxyl, Thio, Nitro und Sulfonamid ausgewählt sind.

17. Verbindung nach Anspruch 16, worin das Heteroaryl-C₁₋₄-alkylen aus Thiophen-2-ylmethyl, Thiophen-3-ylmethyl, Pyrrol-1-ylmethyl, Pyrrol-2-ylmethyl, Pyrrol-3-ylmethyl, Furan-2-ylmethyl, Furan-3-ylmethyl, 2-Thiophen-2-ylethyl, 2-Thiophen-3-yl-ethyl, 2-Furan-2-ylethyl, 2-Furan-3-ylethyl, 2-Pyrrol-1-ylethyl, 2-Pyrrol-2-ylethyl und 2-Pyrrol-3-ylethyl ausgewählt ist.

18. Verbindung nach Anspruch 16, worin das Heteroaryl-C₁₋₄-alkylen aus Thiophen-2-ylmethyl, Thiophen-3-ylmethyl, Pyrrol-1-ylmethyl, Pyrrol-2-ylmethyl, Pyrrol-3-ylmethyl, Furan-2-ylmethyl und Furan-3-ylmethyl ausgewählt ist.

19. Verbindung nach Anspruch 1, worin die Verbindung aus folgenden Verbindungen und pharmazeutisch annehmbaren Salzen, Solvaten und Hydraten davon ausgewählt ist:
4-Fluor-5-methyl-2H-pyrazol-3-carbonsäure;
5-Difluormethyl-4-fluor-2H-pyrazol-3-carbonsäure;
5-(2,2-Difluorethyl)-4-fluor-2H-pyrazol-3-carbonsäure;
5-(1,1-Difluorethyl)-4-fluor-2H-pyrazol-3-carbonsäure;
5-(2,2-Difluorpropyl)-4-fluor-2H-pyrazol-3-carbonsäure;
5-Ethyl-4-fluor-2H-pyrazol-3-carbonsäure;
4-Fluor-5-propyl-2H-pyrazol-3-carbonsäure;
5-Butyl-4-fluor-2H-pyrazol-3-carbonsäure;
4-Fluor-5-pentyl-2H-pyrazol-3-carbonsäure;
4-Fluor-5-hexyl-2H-pyrazol-3-carbonsäure;
4-Fluor-5-heptyl-2H-pyrazol-3-carbonsäure;
4-Fluor-5-octyl-2H-pyrazol-3-carbonsäure;
4-Fluor-5-nonyl-2H-pyrazol-3-carbonsäure;
5-Decyl-4-fluor-2H-pyrazol-3-carbonsäure;
4-Fluor-5-undecyl-2H-pyrazol-3-carbonsäure;
5-Dodecyl-4-fluor-2H-pyrazol-3-carbonsäure;
5-Cyclopropyl-4-fluor-2H-pyrazol-3-carbonsäure und
5-Cyclopropylmethyl-4-fluor-2H-pyrazol-3-carbonsäure.

20. Pharmazeutische Zusammensetzung, umfassend eine Verbindung nach einem der Ansprüche 1 bis 19 in Kombination mit einem pharmazeutisch annehmbaren Träger.

21. Pharmazeutische Zusammensetzung nach Anspruch 20, weiters umfassend ein Mittel, das aus einem α-Glucosidase-Hemmer, einem Aldose-Reductase-Hemmer, Biguanid, einem HMG-CoA-Reductase-Hemmer, einem Squalen-Synthese-Hemmer, Fibrat, einem LDL-Katabolismus-Enhancer, einem Hemmer von Agiotensin-konvertierendem Enzym, einem Insulinsekretionsenhancer und Thiazolidindion ausgewählt ist.

22. Verbindung nach einem der Ansprüche 1 bis 19 zur Verwendung in einem Verfahren zur Behandlung eines menschlichen oder tierischen Körpers durch Therapie.

23. Verbindung nach einem der Ansprüche 1 bis 19 zur Verwendung in einem Verfahren zur Behandlung einer mit dem Stoffwechsel in Zusammenhang stehenden Störung.

24. Verwendung einer Verbindung nach einem der Ansprüche 1 bis 19 zur Herstellung eines Medikaments zur Verwendung bei der Behandlung einer mit dem Stoffwechsel in Zusammenhang stehenden Störung.

25. Verbindung nach einem der Ansprüche 1 bis 19 zur Verwendung bei der Behandlung von Dyslipidämie, Atherosklerose, koronarer Herzkrankheit, Insulinresistenz, Adipositas, beeinträchtigter Glucosetoleranz, Atheromatose, Hypertension, Schlaganfällen, Syndrom X, Herzerkrankungen oder Typ-2-Diabetes.

26. Verwendung einer Verbindung nach einem der Ansprüche 1 bis 19 zur Herstellung eines Medikaments zur Verwendung bei der Behandlung von Dyslipidämie, Atherosklerose, koronarer Herzkrankheit, Insulinresistenz, Adipositas, beeinträchtiger Glucosetoleranz, Atheromatose, Hypertension, Schlaganfällen, Syndrom X, Herzerkrankungen oder Typ-2-Diabetes.

27. Verbindung nach einem der Ansprüche 1 bis 19 zur Verwendung bei der Behandlung von Dyslipidämie, Atherosklerose, koronarer Herzkrankheit, Insulinresistenz oder Typ-2-Diabetes.

28. Verwendung einer Verbindung nach einem der Ansprüche 1 bis 19 zur Herstellung eines Medikaments zur Verwendung in der Behandlung von Dyslipidämie, Atherosklerose, koronarer Herzkrankheit, Insulinresistenz oder Typ-2-Diabetes.

29. Verbindung nach einem der Ansprüche 1 bis 19 zur Verwendung bei der Behandlung von Atherosklerose.

30. Verwendung einer Verbindung nach einem der Ansprüche 1 bis 19 zur Herstellung eines Medikaments zur Verwendung bei der Behandlung von Atherosklerose.

## Revendications

1. Composé sélectionné parmi des composés de Formule (Ia) et leurs sels, solvates, et hydrates pharmaceutiquement acceptables: 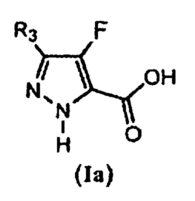 où:
R₃ est cycloalkyle C₃₋₆, alkyle C₁₋₁₂, haloalkyle C₁₋₁₂, cycloalkylC₃₋₆-alkylèneC₁₋₄, arylalkylèneC₁₋₄ ou hétéroaryl-alkylène-C₁₋₄, où ledit aryl-alkylèneC₁₋₄, et ledit hétéroaryl-alkylèneC₁₋₄ peuvent être facultativement substitués par 1 à 5 substituants sélectionnés parmi acyleC₁₋₄, acyloxyC₁₋₄, alcényleC₂₋₄, alcoxyC₁₋₄, alkyleC₁₋₄, alkylcarboxamide C₁₋₄, alkynyle C₂₋₄, alkylsulfonamide C₁₋₄, alkylsulfinyle C₁₋₄, alkylsulfonyle C₁₋₄, alkylthio C₁₋₄, alkyluréyle C₁₋₄, amino, alkylamino C₁₋₄, dialkylamino C₁₋₄, arylsulfonyle, carbo-alcoxyC₁₋₄, carboxamide, carboxy, cyano, cycloakyle C₃₋₆, dialkylcarboxamide C₁₋₄, dialkylsulfonamide C₁₋₄, halogène, haloalcoxyC₁₋₄, haloalkyleC₁₋₄, haloalkylsulfinyle C₁₋₄, haloalkylsulfonyle C₁₋₄, haloalkylthio C₁₋₄, hétérocyclyle, hydroxyle, thio, nitro, oxo-cycloalkyleC₄₋₆, sulfonamide, et acide sulfonique;
où:
"aryle" désigne un radical d'un cycle aromatique contenant 6 à 10 carbones dans le cycle;
"hétéroaryle" désigne un système de cycles aromatiques qui peut être un seul cycle, deux cycles fusionnés ou trois cycles fusionnés où au moins un carbone du cycle est remplacé par un hétéroatome sélectionné parmi O, S et N, où le N est facultativement substitué par H, acyleC₁₋₄, ou alkyleC₁₋₄;
"hétérocyclyle" désigne un cycle de carbone non aromatique à 3, 4, 5, 6 ou 7 membres où un, deux ou trois carbones du cycle sont remplacés par un hétéroatome sélectionné parmi O, S, N, où N est facultativement substitué par H, acyle C₁₋₄ ou alkyle C₁₋₄, et les atomes de carbone du cycle sont facultativement substitués par oxo ou thiooxo; et
"carbo-alcoxyC₁₋₄" désigne un ester alkylique C₁₋₄ d'un acide carboxylique.

2. Composé selon la revendication 1, où R₃ est alkyle C₁₋₁₂.

3. Composé selon la revendication 1, où R₃ est -CH₃, -CH₂CH₃, - (CH₂)₂CH₃, -(CH₂)₃CH₃, -(CH₂)₄CH_{3,} - (CH₂)₅CH₃, -(CH₂)₆CH₃, -(CH₂)₆CH₃, -(CH₂)₇CH₃, -(CH₂)₇CH₃, -(CH₂)₈CH₃, - (CH₂)₉CH₃, - (CH₂) ₁₀CH₃, ou - (CH₂)₁₁CH₃ .

4. Composé selon la revendication 1, où R₃ est -CH₃, -CH₂CH₃, -(CH₂) ₂CH₃, ou -(CH₂)₃CH₃.

5. Composé selon la revendication 1, où R₃ est haloalkyleC₁₋₁₂ .

6. Composé selon la revendication 1, où R₃ est -CHF₂, -CH₂F, -CH₂CF₃, -CH₂CHF₂, -CF₂CH₃, -CH₂CH₂CF₃, -CH₂CF₂CH₃, -CH₂CF₂CF₃, -CH₂CH₂CH₂CHF₂, ou -CH₂CH₂CF₂CH₃.

7. Composé selon la revendication 1, où R₃ est -CF₃, -CF₂CF₃, ou - (CF₂) ₂CF₃.

8. Composé selon la revendication 1, où R₃ est cycloalkyle C₃₋₆.

9. Composé selon la revendication 1, où R₃ est cyclopropyle.

10. Composé selon la revendication 1, où R₃ est cycloalkylC₃₋₆-alkylèneC₁₋₄ facultativement substitué par 1 à 5 substituants sélectionnés parmi alcoxy C₁₋₄, alkyle C₁₋₄, alkylsulfonyle C₁₋₄, alkylthio C₁₋₄, carboxamide, carboxy, cyano, halogène, haloalcoxy C₁₋₄, haloalkyle C₁₋₄, haloalkylsulfonyle C₁₋₄, haloalkylthio C₁₋₄, hydroxyle, thio, nitro, et sulfonamide.

11. Composé selon la revendication 10, où ledit cycloalkyl-C₃₋₆-alkylène-C₁₋₄ est sélectionné parmi cyclopropylméthyle, 2-cylopropyl-éthyle, 1-cyclopropyl-éthyle, 3-cyclopropyl-propyle, 2-cyclopropyl-propyle, 1-cyclopropyl-propyle, 1-cyclopropyl-1-méthyl-éthyle, et 2-cyclopropyl-1-méthyl-éthyle.

12. Composé selon la revendication 11, où ledit cycloalkylC₃₋₆-alkylène C₁₋₄ est cyclopropylméthyle.

13. Composé selon la revendication 1, où R₃ est aryl-alkylèneC₁₋₄ facultativement substitué par 1 à 5 substituants sélectionnés parmi alcoxyC₁₋₄, alkyle C₁₋₄, alkylsulfonyle C₁₋₄, alkylthio C₁₋₄, carboxamide, carboxy, cyano, halogène, haloalcoxyC₁₋₄, haloalkyleC₁₋₄, haloalkylsulfonyle C₁₋₄, haloalkylthio C₁₋₄, hydroxyle, thio, nitro, et sulfonamide.

14. Composé selon la revendication 13, où ledit aryl-alkylène-C₁₋₄ est sélectionné parmi benzyle, phénéthyle, 1-phényl-éthyle, 3-phényl-propyle, 2-phényl-propyle, 1-phényl-propyle, 1-phényl-1-méthyl-éthyle, et 2-phényl-1-méthyl-éthyle.

15. Composé selon la revendication 13, où ledit aryl-alkylène-C₁₋₄ est benzyle.

16. Composé selon la revendication 1, où R₃ est hétéroaryl-alkylène C₁₋₄ facultativement substitué par 1 à 5 substituants sélectionnés parmi alcoxyC₁₋₄, alkyleC₁₋₄, alkylsulfonyleC₁₋₄, alkylthio C₁₋₄, carboxamide, carboxy, cyano, halogène, haloalcoxy C₁₋₄, haloalkyle C₁₋₄, haloalkylsulfonyle C₁₋₄, haloalkylthio C₁₋₄, hydroxyle, thio, nitro, et sulfonamide.

17. Composé selon la revendication 16, où ledit hétéroaryl-alkylène C₁₋₄ est sélectionné parmi thiophén-2-yl-méthyle, thiophén-3-yl-méthyle, pyrrol-1-yl-méthyle, pyrrol-2-yl-méthyle, pyrrol-3-yl-méthyle, furan-2-yl-méthyle, furan-3-yl-méthyle, 2-thiophén-2-yl-éthyle, 2-thiophèn-3-yl-éthyle, 2-furan-2-yl-éthyle, 2-furan-3-yl-éthyle, 2-pyrrol-1-yl-méthyle, 2-pyrrol-2-yl-éthyle, et 2-pyrrol-3-yl-éthyle.

18. Composé selon la revendication 16, où ledit hétéroaryl-alkylène-C₁₋₄ est sélectionné parmi thiophén-2-yl-méthyle, thiophén-3-yl-méthyle, pyrrol-1-yl-méthyle, pyrrol-2-yl-méthyle, pyrrol-3-yl-méthyle, furan-2-yl-méthyle, et furan-3-yl-méthyle.

19. Composé selon la revendication 1, où ledit composé est sélectionné parmi les composés qui suivent et leurs sels, solvates, et hydrates pharmaceutiquement acceptables:
Acide 4-fluoro-5-méthyl-2*H*-pyrazole-3-carboxylique;
Acide 5-difluorométhyl-4-fluoro-2*H*-pyrazole-3-carboxylique;
Acide 5-(2,2-difluoro-éthyl)-4-fluoro-2*H*-pyrazole-3-carboxylique;
Acide 5-(1,1-difluoro-éthyl)-4-fluoro-2*H*-pyrazole-3-carboxylique;
Acide 5-(2,2-difluoro-propyl)-4-fluoro-2*H*-pyrazole-3-carboxylique;
Acide 5-éthyl-4-fluoro-2*H*-pyrazole-3-carboxylique;
Acide 4-fluoro-5-propyl-2*H*-pyrazole-3-carboxylique;
Acide 5-butyl-4-fluoro-2*H*-pyrazole-3-carboxylique;
Acide 4-fluoro-5-pentyl-2*H*-pyrazole-3-carboxylique;
Acide 4-fluoro-5-hexyl-2*H*-pyrazole-3-carboxylique;
Acide 4-fluoro-5-hexyl-2*H*-pyrazole-3-carboxylique;
Acide 4-fluoro-5-octyl-2*H*-pyrazole-3-carboxylique;
Acide 4-fluoro-5-nonyl-2*H*-pyrazole-3-carboxylique;
Acide 5-décyl-4-fluoro-2*H*-pyrazole-3-carboxylique;
Acide 4-fluoro-5-undécyl-2*H*-pyrazole-3-carboxylique;
Acide 5-dodécyl-4-fluoro-2*H*-pyrazole-3-carboxylique;
Acide 5-cyclopropyl-4-fluoro-2*H*-pyrazole-3-carboxylique;
Acide 5-cyclopropylméthyl-4-fluoro-2*H*-pyrazole-3-carboxylique.

20. Composition pharmaceutique comprenant un composé selon l'une quelconque des revendications 1 à 19 en combinaison avec un support pharmaceutiquement acceptable.

21. Composition pharmaceutique selon la revendication 20, comprenant de plus un agent sélectionné parmi un inhibiteur d'α-glucosidase, un inhibiteur d'aldose réductase, le biguanide, un inhibiteur de HMG-CoA réductase, un inhibiteur de la synthèse du squalène, le fibrate, un activateur du catabolisme de LDL, un inhibiteur de l'angine de conversion de l'angiotensine, un activateur de la sécrétion de l'insuline et de la thiazolidinedione.

22. Composé selon l'une quelconque des revendications 1 à 19 à utiliser dans une méthode de traitement du corps humain ou animal par thérapie.

23. Composé selon l'une quelconque des revendications 1 à 19 à utiliser dans une méthode de traitement d'un trouble en rapport avec le métabolisme.

24. Utilisation d'un composé selon l'une quelconque des revendications 1 à 19 dans la fabrication d'un médicament à utiliser dans le traitement d'un trouble en rapport avec le métabolisme.

25. Composé selon l'une quelconque des revendications 1 à 19 à utiliser dans le traitement de la dyslipidémie, de l'athérosclérose, d'une maladie des coronaires, de la résistance à l'insuline, de l'obésité, de la tolérance entravée au glucose, d'une maladie athéromateuse, de l'hypertension, d'une attaque cérébrale, du syndrome X, d'une maladie cardiaque, ou du diabète type 2.

26. Utilisation d'un composé selon l'une quelconque des revendications 1 à 19 dans la fabrication d'un médicament à utiliser dans le traitement de la dyslipidémie, de l'athérosclérose, d'une maladie de coronaires, de la résistance à l'insuline, de l'obésité, d'une tolérance entravée au glucose, d'une maladie athéromateuse, de l'hypertension, d'une congestion cérébrale, du syndrome X, d'une maladie cardiaque ou du diabète type 2.

27. Composé selon l'une quelconque des revendications 1 à 19 à utiliser dans le traitement de la dyslipidémie, de l'athérosclérose, d'une maladie des coronaires, d'une résistance à l'insuline, ou du diabète type 2.

28. Utilisation d'un composé selon l'une quelconque des revendications 1 à 19 dans la fabrication d'un médicament à utiliser dans le traitement de la dyslipidémie, de l'athérosclérose, d'une maladie des coronaires, de la résistance à l'insuline ou du diabète type 2.

29. Composé selon l'une quelconque des revendications 1 à 19 à utiliser dans le traitement de l'athérosclérose.

30. Utilisation d'un composé selon l'une quelconque des revendications 1 à 19 dans la fabrication d'un médicament à utiliser dans le traitement de l'athérosclérose.


### SEQUENCE LISTING

<110> Arena Pharmaceuticals, Inc.
   Semple, Graeme
   Averbuj, Claudia
   Skinner, Philip
   Gharbaoui, Tawfik
   Shin, Young Jun
<120> HYDROXYPYRAZOLES AND METHODS OF PROPHYLAXIS OR TREATMENT OF METABOLIC-RELATED DISORDERS THEREOF
<130> 29.WO1
<150> 60/416,193
   <151> 2002-10-04
<150> 60/417,120
   <151> 2002-10-07
<160>
<170> PatentIn version 3.2
<210> 1
   <211> 1092
   <212> DNA
   <213> Homo sapien
<400> 1
   atgaatcggc accatctgca ggatcacttt ctggaaatag acaagaagaa ctgctgtgtg    60
   ttccgagatg acttcattgt caaggtgttg ccgccggtgt tggggctgga gtttatcttc   120
   gggcttctgg gcaatggcct tgccctgtgg attttctgtt tccacctcaa gtcctggaaa   180
   tccagccgga ttttcctgtt caacctggca gtggctgact ttctactgat catctgcctg   240
   cccttcctga tggacaacta tgtgaggcgt tgggactgga agtttgggga catcccttgc   300
   cggctgatgc tcttcatgtt ggctatgaac cgccagggca gcatcatctt cctcacggtg   360
   gtggcggtag acaggtattt ccgggtggtc catccccacc acgccctgaa caagatctcc   420
   aatcggacag cagccatcat ctcttgcctt ctgtggggca tcactattgg cctgacagtc   480
   cacctcctga agaagaagat gccgatccag aatggcggtg caaatttgtg cagcagcttc   540
   agcatctgcc ataccttcca gtggcacgaa gccatgttcc tcctggagtt cttcctgccc   600
   ctgggcatca tcctgttctg ctcagccaga attatctgga gcctgcggca gagacaaatg   660
   gaccggcatg ccaagatcaa gagagccatc accttcatca tggtggtggc catcgtcttt   720
   gtcatctgct tccttcccag cgtggttgtg cggatccgca tcttctggct cctgcacact   780
   tcgggcacgc agaattgtga agtgtaccgc tcggtggacc tggcgttctt tatcactctc   840
   agcttcacct acatgaacag catgctggac cccgtggtgt actacttctc cagcccatcc   900
   tttcccaact tcttctccac tttgatcaac cgctgcctcc agaggaagat gacaggtgag   960
   ccagataata accgcagcac gagcgtcgag ctcacagggg accccaacaa aaccagaggc  1020
   gctccagagg cgttaatggc caactccggt gagccatgga gcccctctta tctgggccca  1080
   acctctcctt aa                                                      1092
<210> 2
   <211> 363
   <212> PRT
   <213> Homo sapien
<400> 2